# EUROPEAN PATENT SPECIFICATION

(11) **EP 0 921 125 B1**
(45) Date of publication and mention of the grant of the patent: **30.01.2002**
(21) Application number: 98122511.3
(22) Date of filing: 27.11.1998
(51) Int. Cl.: C07D 471/10, A61K 31/44

(54) **1,3,8-Triazaspiro[4,5]decan-4-on derivatives**
1,3,8-Triazaspiro[4,5]decan-4-on-derivate
DERIVES DE LA 1,3,8-TRIAZASPIRO[4,5]DECAN-4-ONE

(30) Priority: 05.12.1997 EP 97121427
(43) Date of publication of application: 09.06.1999
(73) Proprietor: F. HOFFMANN-LA ROCHE AG, 4070 Basel (CH)
(72) Inventor: Adam, Geo, 79650 Schopfheim (DE); Cesura, Andrea, 4056 Basle (CH); Galley, Guido, 79618 Rheinfelden (DE); Jenck, Francois, 68400 Riedisheim (FR); Röver, Stephan, 79594 Inzlingen (DE); Wichmann, Jürgen, 79585 Steinen (DE)
(74) Representative: Poppe, Regina

(56) References cited:
- EP-A- 0 035 902
- EP-A- 0 856 514

## Description

The present invention relates to compounds of the general formula 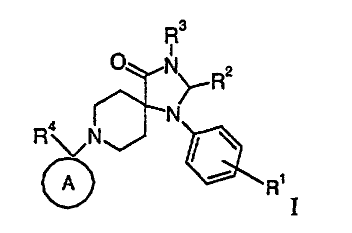 wherein
- R¹: is hydrogen, (C₁₋₆)-alkyl, halogen, (C₁₋₆)-alkoxy, trifluoromethyl, (C₁₋₆)-alkyl-phenyl or (C₅₋₇)-cycloalkyl;
- R²: is hydrogen, (C₁₋₆)-alkyl, phenyl or (C₁₋₆)-alkyl-phenyl;
- R³: is hydrogen, (C₁₋₆)-alkyl, benzyl, (C₁₋₆)-alkyl-phenyl, (C₁₋₆)-alkyl-diphenyl, triazinyl, cyanomethyl, (C₁₋₆)-alkyl-piperidinyl, (C₁₋₆)-alkyl-naphthyl, (C₅-₇)-cycloalkyl, (C₁₋₆)-alkyl-(C₅₋₇)-cycloalkyl, (C₁₋₆)-alkyl-pyridinyl, (C₁₋₆)-alkyl-morpholinyl, (C₁₋₆)-alkyl-dioxolanyl, (C₁₋₆)-alkyl-oxazolyl or (C₁₋₆)-alkyl-2-oxo-oxazolidinyl and wherein the ring systems may be substituted by additional (C₁₋₆)-alkyl, (C₁₋₆)-alkoxy, trifluoromethyl or phenyl, or -(CH₂)ₙC(O)O-(C₁₋₆)- alkyl, (CH₂)ₙC(O)NH₂, -(CH₂)ₙC(O)N[(C₁₋₆)-alkyl]₂, (CH₂)ₙOH or -(CH₂)ₙC(O)NHCH₂C₆H₅;
- R⁴: is hydrogen, (C₁₋₆)-alkyl or nitrilo;
- A: is a ring system, consisting of
(a) (C₅₋₁₅)-cycloalkyl, which may be in addition to R⁴ optionally substituted by (C₁₋₆)-alkyl, trifluoromethyl, (C₅₋₇)-cycloalkyl, spiro-undecan-alkyl or by 2-norbornyl, or is one of the following groups
(b) 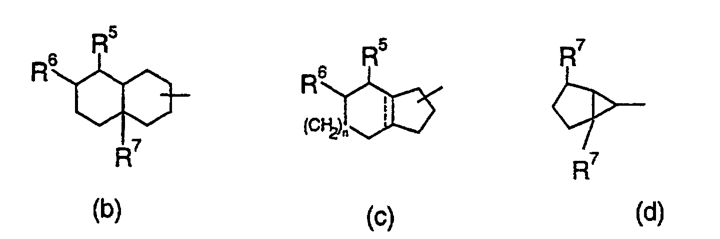 dodecahydro-acenaphthylen-lyl (e), bicyclo[6.2.0]dec-9-yl (f) and bicyclononan-9-yl (g);
and wherein
- R⁵ and R⁶: are hydrogen, (C₁₋₆)-alkyl or taken together and with the carbon atoms to which they are attached form a phenyl ring;
- R⁷: is hydrogen or (C₁₋₆)-alkyl;
the dotted line represents an optional bond and
- n: is 1 to 4;
and to pharmaceutically acceptable acid addition salts thereof.

The compounds of formula I and their salts are characterized by valuable therapeutic properties. It has surprisingly been found that the compounds of the present invention are agonists and/or antagonists of the Orphanin FQ (OFQ) receptor. Consequently they will be useful in the treatment of memory and attention deficits, psychiatric, neurological and physiological disorders, especially, but not limited to, amelioration of symptoms of anxiety and stress disorders, depression, trauma, memory loss due to Alzheimer's disease or other dementias, epilepsy and convulsions, acute and/or chronic pain conditions, symptoms of addictive drug withdrawal, control of water balance, Na⁺ excretion, arterial blood pressure disorders and metabolic disorders such as obesity.

OFQ, a seventeen amino-acid-long peptide
(F-G-G-F-T-G-A-R-K-S-A-R-K-L-A-N-Q), has been isolated from rat brain and is a natural ligand to a G-protein coupled receptor (OFQ-R), found at high levels in brain tissue.

OFQ exhibits agonistic action at the OFQ-R both in vitro and in vivo.

Julius (Nature 377,476, [1995]) discusses the discovery of OFQ noting that this peptide shares greatest sequence similarity with dynorphin A, an established endogenous ligand for opioid receptors. OFQ inhibits adenylate cyclase in CHO(LC 132⁺) cells in culture and induces hyperalgesia when administered intra-cerebroventricularly to mice. The pattern of results indicate that this heptadecapeptide is an endogenous agonist of the LC 132 receptor and it appears to have pro-nociceptive properties. It was described that when injected intra-cerebroventricularly in mice, OFQ slowed down locomotive activity and induced hyperalgesia. It was concluded that OFQ may act as a brain neurotransmitter to modulate nociceptive and locomotive behavior.

Objects of the present invention are the compounds of formula I and pharmaceutically acceptable addition salts thereof, racemic mixtures and their corresponding enantiomers, the preparation of the above-mentioned compounds, medicaments containing them and their manufacture as well as the use of the above-mentioned compounds in the control or prevention of illnesses, especially of illnesses and disorders of the kind referred to earlier, or in the manufacture of corresponding medicaments.

The following definitions of the general terms used in the present description apply irrespective of whether the terms in question appear alone or in combination.

As used herein, the term "(C₁₋₆)-alkyl" denotes a straight- or branched-chain alkyl group containing from 1 to 6 carbon atoms, for example, methyl, ethyl, propyl, isopropyl, n-butyl, i-butyl, 2-butyl, t-butyl and the like. Preferred (C₁₋₆)-alkyl groups are groups with 1 - 4 carbon atoms.

The term "cycloalkyl" denotes a saturated carbocydic group containing from 5 - 15 carbon atoms, preferred are cyclohexyl, cyclodecyl and cyclononyl.

The term "halogen" denotes chlorine, iodine, fluorine and bromine.

The term "(C₁₋₆)-alkoxy" denotes a group wherein the alkyl residues is as defined above, and which is attached via an oxygen atom.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

Exemplary preferred are compounds, in which A is a unsubstituted decahydronaphthalen group (formula b) and R¹ - R³ are hydrogen or R³ is methyl and R¹ and R² are hydrogen, for example the following compounds:
8-(Decahydro-naphthalen-2-yl)-1-phenyl-1,3,8-triaza-spire[4.5]decor-4-one,
8-(Decahydro-naphthalen-2-yl)-3-methyl-1-phenyl-1,3,8-triaza-spiro [4.5]decan-4-one,
mixture of (2RS,4aRS,8aSR)- and (2SR,4aRS,8aSR)-8-(decahydro-naphthalen-2-yl)-1-phenyl-1,3,8-triaza-spiro[4.5]decan-4-one or
(2RS,4aSR,8aRS)-8-(Decahydro-naphthalen-2-yl)- 1-phenyl-1,3,8-triazaspiro[4.5]decan-4-one.

Further preferred are compounds, in which A is cyclohexyl, optionally substituted by methyl or isopropyl, cyclodecyl or cyclononyl (formula a) and R¹-R³ are hydrogen.

Examples of such compounds are
cis-8-(4-Methyl-cyclohexyl)-1-phenyl-1,3,8-triaza-spiro[4.5]decan-4-one,
8-Cyclodecyl-1-phenyl-1,3,8-triaza-spiro[4.5] decan-4-one
8-Cyclononyl-1-phenyl-1,3,8-triaza-spiro[4.5]decan-4-one or
cis-8-(4-Isopropyl-cyclohexyl)-1-phenyl-1,3,8-triaza-spire[4.5]decan-4-one.

Preferred are further compounds, in which A is cyclodecyl, (formula a) and R¹ is hydrogen or methyl, R² is hydrogen or phenyl and R³ is hydrogen or acetonitrile.

Examples of such compounds are
(R,S)-8-Cyclodecyl-1-(3-methyl-phenyl)-2-phenyl-1,3,8-triazaspiro[4,5]decan-4-one or
8-Cyclodecyl-4-oxo-1-phenyl-1,3,8-triaza-spiro[4,5] dec-3-yl)-acetonitrile.

Further preferred are compounds, in which A is octahydro-inden (formula c) and R¹-R³ are hydrogen, for example the following compound:
8-(cis-Octahydro-inden-2-yl)-1-phenyl-1,3,8-triaza-spiro[4.5]decan-4-one.

Further preferred is the following compound:
8-(cis-Bicyclo[6.2.0]dec-9-yl)-1-phenyl-1,3,8-triaza-spiro[4,5]decan-4-one.

The present compounds of formula I and their pharmaceutically acceptable salts can be prepared by methods known in the art, for example, by processes described below, which comprise
a) reductively aminating a compound of formula 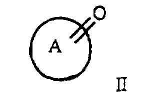 with a compound of formula 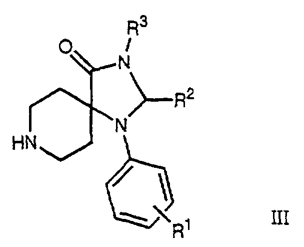 or, if desired, adding KCN to the mixture of a compound of formula II and III to get a compound of formula I, wherein R⁴ is hydrogen or nitrilo, respectively, and wherein A and R¹-R³ have the significances given above, or
b) cyclizing and reducing a compound of formula 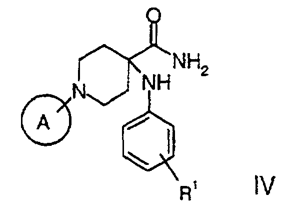 to give a compound of formula 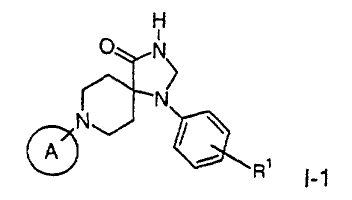 wherein A and R¹ have the significances given above, or
c) reacting a compound of formula 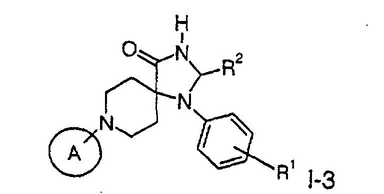 with a compound of formula

   R³X

   wherein R³ is described as above, but different from hydrogen and X is halogen, to give a compound of formula 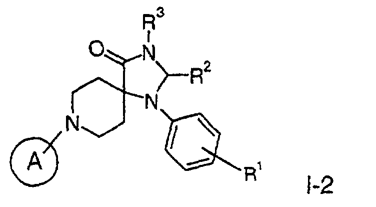 or
d) alkylating or benzylating a compound of formula I, wherein R³ is hydrogen, and, if desired,
e) converting a racemic mixture into its enantiomeric component thus obtaining optically pure compounds, and/or,if desired,
f) converting the compound of formula I obtained into a pharmaceutically acceptable acid addition salt.

In accordance with process variant a) the reductive amination of a keto compound of formula II with an amine of formula III is carried out in conventional manner in a solvent, such as toluene, tetrahydrofuran (THF), methanol or ethanol, or in a mixture of THF with a suitable alcohol, and in the present of a reducing agent such as Na-cyanoborhydride, or in the presence of KCN, respectively.

Another method for a reductive amination is described in J. Org. Chem., 55, 2552-54, 1990. In accordance with this variant the reaction is carried out by reaction of an amine with a ketone in the presence of TI-(IV)-isopropoxide and Na-cyanoborohyride.

The cyclisation in accordance with process step b) can be carried out in conventional manner, for example with formamide at a temperature of about 200 °C or with formic acid ortho esters at a temperature of about 150 °C. Unsaturated products can be converted to the saturated compounds by reduction, for example with sodium cyanoborohydride.

A compound of formula I can further be prepared by reacting a compound of formula I-1 with a compound of formula R³X in accordance with reaction step c). This reaction is carried out in a suitable solvent, such as DMF, and in the presence of sodium hydride at room temperature. Suitable halogenides of formula R₃X are chlorides, bromides and iodides, such as methyl iodide, bromomethyl acetate, N,N-dimethyl-chloroacetamide or ethyl-5-bromovaleriate. The reaction time is about 18 h.

In accordance with process variant d) a compound of formula I, wherein R³ is hydrogen, can be alkylated or benzylated in conventional manner, for example in the presence of a corresponding alkyl- or benzyl halogenide, such as methyliodide, ethyliodide, benzylbromide, 3-phenyl-1-propylbromide or 3,3-diphenyl-1-propylbromide. This reaction is carried out in the presence of a metal hydride, such as sodium hydride at a temperature of about 60-100 °C.

Racemic mixtures can be converted into its enantiomeric components in conventional manner, for example by preparative HPLC.

The salt formation in accordance with variant f) is effected at room temperatures in accordance with methods which are known per se and which are familiar to any person skilled in the art. Not only salts with inorganic acids, but also salts with organic acids come into consideration. Hydrochlorides, hydrobromides, sulphates, nitrates, citrates, acetates, maleates, succinates, methanesulphonates, p-toluenesulfonates and the like are examples of such salts.

The compounds of formulae II and III which are used as starting materials are known compounds or can be prepared by methods known per se.

For example, the ketons of formula II can be prepared by methods, described in Beilstein H7, EI7, EII7, EIII7 and EIV7. Compounds of formula III are described, for example, in US 3,238,216, US 3,161,644, US 3,155,670 and US 3,155,669.

Schemes 1 and 4 describe the reductive amination in accordance with process variant a). 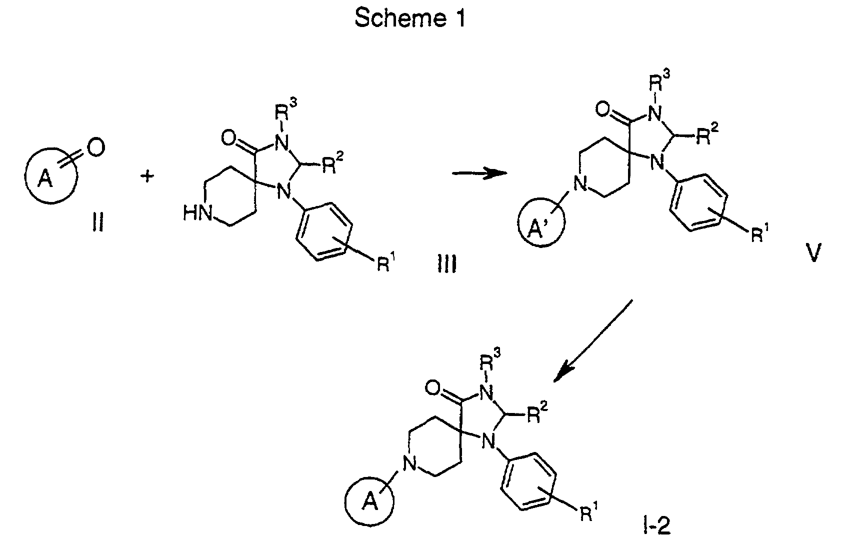 wherein A' is a compound of group A having a partially unsaturated ring (one double bond) and A and R¹-R³ have the significances given above.
Scheme 2 shows a process for the preparation of compounds of formula I-1. 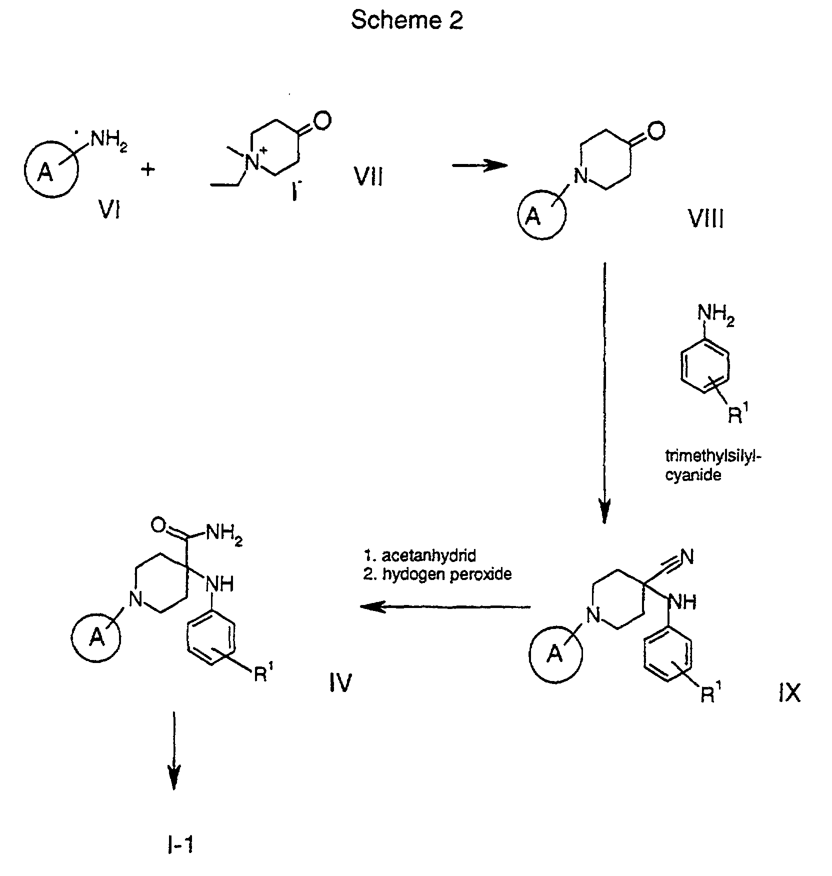 R¹ and A have the significances given above.
The starting materials of formulae VI and VII are known compounds or can be prepared according to methods known in the art, for example compounds of formula VI can be prepared in accordance with Beilstein H12, EI12, EII12, EIII12 and EIV12. Compounds of formula VII can be prepared by methods described in Tschaen et al., J.Org.Chem., 60, 4324 (1995).
In scheme 3 it is described the cyclization of compounds of formula IV to a compound of formula X and then to a compound of formula I-2. In this scheme the substituents are as described above. The starting materials of formula IV are known compounds or can be prepared by methods known in the art. 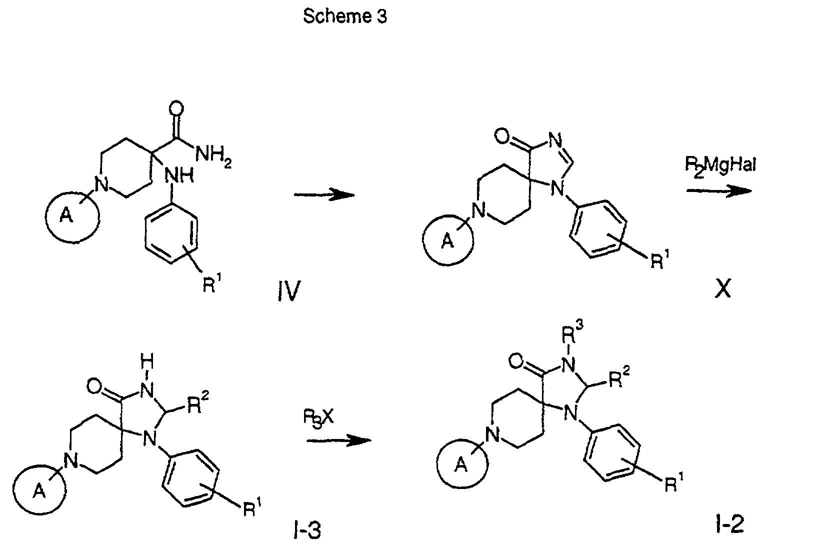 In the following scheme 4 is described the process for preparation of compounds of formula I, wherein A is cyclohexyl, optionally substituted by (C₁₋₆) alkyl, C₅₋₇-cycloalkyl, spiro-undecan-alkyl or 2-norbonyl and R⁴ in this scheme is (C₁₋₆) alkyl.

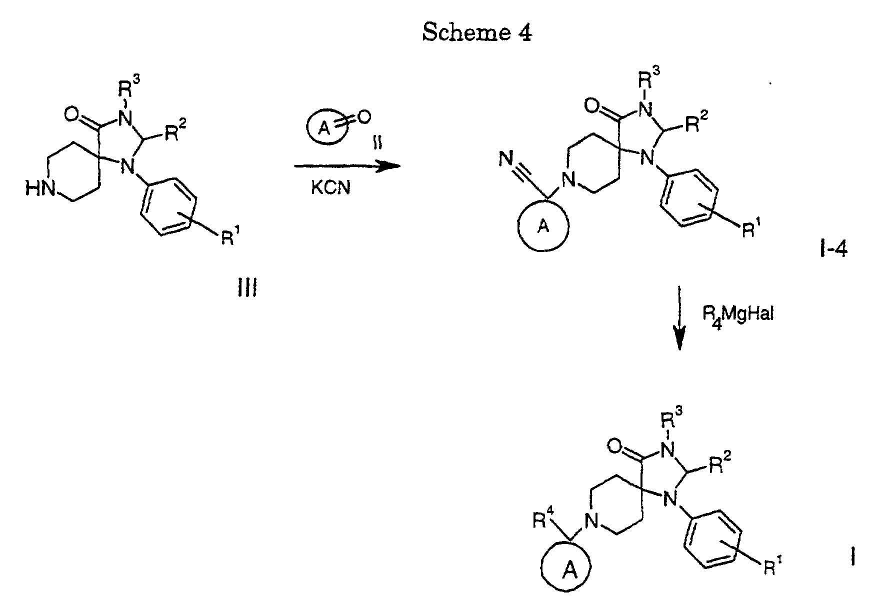

As mentioned earlier, the compounds of formula I and their pharmaceutically usuable addition salts possess valuable pharmacodynamic properties. It has been found that the compounds of the present invention are agonists and/or antagonists of the OFQ receptor and have effects in animal models of psychiatric, neurological and physiological disorders, such as anxiety, stress disorders, depression, trauma, memory loss due to Alzheimer's disease or other dementias, epilepsy and convulsions, acute and/or chronic pain conditions, symptoms of addictive drug withdrawal, control of water balance, Na⁺ excretion, arterial blood pressure disorders and metabolic disorders such as obesity.

The compounds were investigated in accordance with the tests given hereinafter:

### Methods of OFQ-R Binding Assay

### Cell Culture

HEK-293 cells adapted to suspension growth (293s) were cultured in HL medium plus 2% FBS. The cells were transfected with the rat OFQ receptor cDNA (LC132), FEBS Lett. 347, 284-288, 1994, cloned in the expression vector pCEP4 (Invitrogen, SanDiego, CA, USA) using lipofectin (Life Technologies, Bethesda, MD, USA). Transfected cells were selected in the presence of hygromycin (1000 U/ml) (Calbiochem, SanDiego, CA, USA). A pool of resistant cells was tested for OFQ-R expression by binding of [³H]-OFQ (Amersham PLC, Buckinghamshire, England). These cells (293s-OFQ-R) were expanded for large scale culture and membrane preparation.

### Membrane preparation

293s-OFQ-R cells were harvested by centrifugation, washed 3 times with phosphate buffered saline (PBS) before resuspension in buffer A (50 mM Tris-HCl, pH 7.8, 5 mM MgCl₂, 1 mM EGTA) and disruption with a tissue homogenizer (30 seconds, setting 4, Pt 20, Kinematica, Kriens-Lucern, Switzerland). A total membrane fraction was obtained by centrifugation at 49,000 x g at 4°C. This procedure was repeated twice and the pellet was resuspended in buffer A. Aliquots were stored at -70°C and protein concentrations were determined using the BCA™ Protein Assay Reagent (Pierce, Rockford, IL) following the manufacturer's recommendations.
Binding Assays [³H]-OFQ competition studies were carried out with 77 µg membrane protein in a final assay volume of 0.5 ml buffer A plus 0.1% BSA and 0.01% bacitracin (Boehringer-Mannheim, Mannheim, Germany) for one hour at room temperature. 50 nM unlabeled OFQ was used to define the non-specific binding. The assays were terminated by filtration through Whatman GF/C filters (Unifilter-96, Canberra Packard S.A., Zurich, Switzerland) pretreated with 0.3% polyethylenimine (Sigma, St. Louis, MO, USA) and 0.1% BSA (Sigma) for 1 hour. The filters were washed 6 times with 1 ml of ice bold 50 mM Tris-HCl pH 7.5. The retained radioactivity was counted on a Packard Top-Count microplate scintillation counter after addition of 40 µl of Microscint 40 (Canberra Packard). The effects of compounds were determined using at least 6 concentrations in triplicate, and determined twice. IC₅₀ values were determined by curve fitting and these values were converted to Kᵢ values by the method of Cheng and Prusoff, Biochem. Pharmacol., 22, 3099, 1973.

The affinity to the OFQ-receptor, given as pKᵢ, is in the scope of 6,2 to 10,0. For example, the pKᵢ -values of Examples 13 and 21 are 8,4 and 9,5, respectively.
- 13: Mixture ofcis- and bans-1-phenyl-8-(4-propyl-cyclohexyl)-1,3,8-triaza-spiro[4.5]decan-4-one
- 21: 8-(Decahydro-naphthalen-2-yl)-3-methyl-1-phenyl-1,3,8-triaza-spiro[4.5]decan-4-one

The compounds of formula I as well as their pharmaceutically usable acid addition salts can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The compounds of formula I and their pharmaceutically usable acid addition salts can be processed with pharmaceutically inert, inorganic or organic excipients for the production of tablets, coated tablets, dragees and hard gelatine capsules. Lactose, corn starch or derivatives thereof, talc, stearic acid or its salts etc can be used as such excipients e.g. for tablets, dragées and hard gelatine capsules.

Suitable excipients for soft gelatine capsules are e.g. vegetable oils, waxes, fats, semi-solid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semi-liquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary within wide limits and will, of course, be fitted to the individual requirements in each particular case. In general, in the case of oral administration a daily dosage of about 10 to 1000 mg per person of a compound of general formula I should be appropriate, although the above upper limit can also be exceeded when necessary.

The following Examples illustrate the present invention without limiting it. All temperatures are given in degrees Celsius.

### Example 1

### 8-Cyclohexyl-1-phenyl-1,3,8-triaza-spiro[4.5]decan-4-one hydrochloride (1:1)

Cyclohexanone (4.3 mmol) was dissolved in toluene (50 ml). 1-Phenyl-1,3,8-triazaspiro[4.5]decan-4-one (4.3 mmol) and molecular sieves (3.3 g) were added and the mixture was refluxed for 18 h. After cooling the molecular sieves were removed by filtration and washed with methylenchloride. Evaporation of the filtrate yielded a residue which was dissolved in THF (45 ml) and ethanol (5 ml). Sodium cyanoborohydride (4.3 mmol) was added to the solution and the pH was adjusted to 4. The mixture was stirred for 3 h at room temperature. Water and ethylacetate was added and the organic phase was washed with 2 N sodium hydroxide and brine. The organic phase was dried with Na₂SO₄ and concentrated. Chromatography on silica gel (methylenchloride/methanol, 98:2) yielded the desired product which was crystallized as its HCl-salt from ethylacetate/methanol. 0.96 g (64%) 8-cydohexyl-1-phenyl-1,3,8-triaza-spiro[4.5]decan-4-one hydrochloride (1:1) as a colorless solid, m.p. > 250 °C and MS: m/e = 313 (M⁺).

### Example 2

### cis-8-(4-tert-Butyl-cyclohexyl)-1-phenyl-1,3,8-triaza-spiro[4.5]decan-4-one hydrochloride (1:1)

The tide compound, m.p. > 250 °C and MS: m/e = 370 (M+H⁺) was prepared in accordance with the general method of example 1 from 4-(1,1-dimethylethyl)-cyclohexanone and 1-phenyl-1,3,8-triazaspiro[4.5]decan-4-one.

### Example 3

### Mixture of (1RS,3RS)- and (1RS,3SR)-8-(3-methyl-cyclohexyl)-1-phenyl-1,3,8-triaza-spiro[4.5]decan-4-one hydrochloride (1:1)

The tide compound, m.p. > 250 °C and MS: m/e = 328 (M+H⁺) was prepared in accordance with the general method of example 1 from 3-methyl-cyclohexanone and 1-phenyl- 1,3,8-triazaspiro[4.5]decan-4-one.

### Example 4

### Mixture of cis- and trans-8-(4-methyl-cyclohexyl)-1-phenyl-1,3,8-triaza-spiro[4.5]decan-4-one hydrochloride (1:1)

The title compound, m.p. > 250 °C and MS: m/e = 328 (M+H⁺) was prepared in accordance with the general method of example 1 from 4-methyl-cyclohexanone and 1-phenyl-1,3,8-triazaspiro [4.5] decan-4-one.

### Example 5

### trans-8-(4-tert-Butyl-cyclohexyl)-1-phenyl-1,3,8-triaza-spiro[4.5]decan-4-one hydrochloride (1:1)

The tide compound, m.p. > 250 °C and MS: m/e = 370 (M+H⁺) was prepared in accordance with the general method of example 1 from 4-(1,1-dimethylethyl)-cyclohexanone and 1-phenyl-1,3,8-triazaspiro [4.5] decan-4-one.

### Example 6

### 1-Phenyl-8- (3,3,5,5-tetramethyl-cyclohexyl)-1,3,8-triaza-spiro[4.5]decan-4-one hydrochloride (1:1)

The tide compound, m.p. > 250 °C and MS: m/e = 370 (M+H⁺) was prepared in accordance with the general method of example 1 from 3,3,5,5-tetramethyl-cyclohexanone and 1-phenyl-1,3,8-triazaspiro[4.5]decan-4-one.

### Example 7

### Mixture of (1RS,2RS)- and (1RS,2SR)-8-(2-methyl-cyclohexyl)-1-phenyl-1,3,8-triaza-spiro[4.5]decan-4-one hydrochloride (1:1)

2-Methyl-cyclohexanone (4.5 mmol) was dissolved in THF (10 ml). 1-Phenyl-1,3,8-triazaspiro[4.5]decan-4-one (4.5 mmol) and tetraisopropyl-orthotitanate (4.5 mmol) were added and the mixture was stirred for 18 h at room temperature. Evaporation yielded a residue which was dissolved in ethanol (7 ml). Sodium cyanoborohydride (4.3 mmol) was added to the solution and the mixture was stirred for 18 h at room temperature. Water and ethylacetate was added and the organic phase was washed with 2 N sodium hydroxide and brine. The organic phase was dried with Na₂SO₄ and concentrated. Chromatography on silica gel (dichloromethane/methanol, 98:2) yielded the desired product which was crystallized as its HCl-salt from ethylacetate/ethanol. 0.34 g (21%) of a mixture of (1RS,2RS)- and (1RS,2SR)-8-(2-methyl-cyclohexyl)-1-phenyl-1,3,8-triaza-spiro[4.5]decan-4-one hydrochloride (1:1) as a colorless solid, m.p. > 250 °C and MS: m/e = 327 (M⁺).

### Example 8

### 8-(Decahydro-naphthalen-2-yl)-1-phenyl-1,3,8-triaza-spiro[4.5]decan-4-one hydrochloride (1:1) (mixture of the diast. racemates)

The title compound, m.p. > 250 °C and MS: m/e = 368 (M+H⁺) was prepared in accordance with the general method of example 1 from octahydro-2(1H)-naphthalenone and 1-phenyl-1,3,8-triazaspiro[4.5]decan-4-one.

### Example 9

### Mixture of (1R,3R)- and (1S,3R)-8-(3-methyl-cyclohexyl)-1-phenyl-1,3,8-triaza-spiro[4.5]decan-4-one hydrochloride (1:1)

The title compound, m.p. > 250 °C and MS: m/e = 328 (M+H⁺) was prepared in accordance with the general method of example 1 from (R)-3-methyl-cydohexanone and 1-phenyl-1,3,8-triazaspiro [4.5] decan-4-one.

### Example 10

### Mixture of (1RS,4aRS,8aSR)- and (1SR,4aRS,8aSR)-8-(Decahydro-naphthalen-1-yl)-1-phenyl-1,3,8-triaza-spire[4.5]decor-4-one hydrochloride (1:1)

The title compound, m.p. > 250 °C and MS: m/e = 367 (M⁺) was prepared in accordance with the general method of example 7 from trans-octahydro-2(1H)-naphthalenone and 1-phenyl-1,3,8-triazaspiro[4.5]decan-4-one.

### Example 11

### Mixture of (1RS,3RS)- and (1RS,3SR)-8-(3-tert-butyl-cyclohexyl)-1-phenyl-1,3,8-triaza-spiro[4.5]decan-4-one hydrochloride (1:1)

The title compound, m.p. > 250 °C and MS: m/e = 369 (M⁺) was prepared in accordance with the general method of example 1 from 3-(1,1-dimethylethyl)-cyclohexanone and 1-phenyl- 1,3,8-triazaspiro [4.5] decan-4-one.

### Example 12

### Mixture of (1RS,5RS)-and(1RS,5SR)-1-phenyl-8-(3,3,5-trimethyl-cyclohexyl)-1,3,8-triaza-spiro[4.5]decan-4-one hydrochloride (1:1)

The title compound, m.p. > 250 °C and MS: m/e = 355 (M⁺) was prepared in accordance with the general method of example 1 from 3,3,5-trimethyl-cyclohexanone and 1-phenyl-1,3,8-triazaspiro[4.5]decan-4-one.

### Example 13

### Mixture of cis- and trans 1-phenyl-8-(4-propyl-cyclohexyl)-1,3,8-triaza-spiro[4.5]decan-4-one hydrochloride (1:1)

The title compound, m.p. > 250 °C and MS: m/e = 356 (M+H⁺) was prepared in accordance with the general method of example 1 from 4-propyl-cyclohexanone and 1-phenyl-1,3,8-triazaspiro[4.5]decan-4-one.

### Example 14

### 1:1 Mixture of (2R,4aS,8aR)- and (2S,4aS,8aR)-8-(4a-methyl-decahydro-naphthalen-2-yl)-1-phenyl-1,3,8-triaza-spiro[4.5]decan-4-one hydrochloride (1:1)

The title compound, m.p. > 250 °C and MS: m/e = 382 (M+H⁺) was prepared in accordance with the general method of example 1 from (4aS-cis)-octahydro-4a-methyl-2(1H)-naphthalenone and 1-phenyl-1,3,8-triazaspiro[4.5]decan-4-one.

### Example 15

### 1:1 Mixture of (2R,4aR,8aS)- and (2S,4aR,8aS)-8-(4a-methyl-decahydro-naphthalen-2-yl)-1-phenyl-1,3,8-triaza-spiro[4.5]decan-4-one hydrochloride (1:1)

The title compound, m.p. > 250°C and MS: m/e = 382 (M+H⁺) was prepared in accordance with the general method of example 1 from (4aR-cis)-octahydro-4a-methyl-2(1H)-naphthalenone and 1-phenyl-1,3,8-triazaspiro[4.5]decan-4-one.

### Example 16

### (2S,4aR,8aS)-8-(4a-Methyl-decahydro-naphthalen-2-yl)-1-phenyl-1,3,8-triaza-spiro[4.5]decan-4-one

The tide compound, NMR (CDCl₃): 0.96 (s, 3H), 1.8-1.0 (m, 17 H), 2.4 (m, 1H), 2.62 (m, 2H), 2.85 (m, 2H), 3.05 (t, 2H), 4.72 (s, 2H), 6.46 (bs, 1H), 6.89 (m, 3H), 7.27 (t, 2H) was isolated by preparative HPLC of the mixture obtained in example 15.

### Example 17

### (2R,4aR,8aS)-8-(4a-Methyl-decahydro-naphthalen-2-yl)-1-phenyl-1,3,8-triaza-spiro[4.5]decan-4-one

The title compound, NMR (CDCl₃): 0.97 (s, 3H), 1.8-1.0 (m, 17 H), 2.58 (m, 3H), 2.84 (m, 2H), 3.02 (m, 2H), 4.72 (s, 2H), 6.40 (bs, 1H), 6.92 (m, 3H), 7.27 (t, 2H) was isolated by preparative HPLC of the mixture obtained in example 15.

### Example 18

### (2S,4aS,8aR)-8-(4a-Methyl-decahydro-naphthalen-2-yl)-1-phenyl-1,3,8-triaza-spiro[4.5]decan-4-one

The title compound, NMR (CDCl₃): 0.97 (s, 3H), 1.8-1.0 (m, 17H), 2.4 (m, 1H), 2.60 (m, 2H), 2.83 (m, 2H), 3.05 (t, 2H), 4.73 (s, 2H), 6.58 (bs, 1H), 6.87 (t, 1H), 6.94 (d, 2H), 7.27 (t, 2H) was isolated by preparative HPLC of the mixture obtained in example 14.

### Example 19

### 1:1 Mixture of (2R,4aS,8aS)- and (2S,4aS,8aS)-8-(4a-methyl-decahydro-naphthalen-2-yl)-1-phenyl-1,3,8-triaza-spiro[4.5]decan-4-one hydrochloride (1:1)

The title compound, m.p. > 250 °C and MS: m/e = 382 (M+H⁺) was prepared in accordance with the general method of example 1 from (4aS-trans)-octahydro-4a-methyl-2(1H)-naphthalenone and 1-phenyl-1,3,8-triazaspiro[4.5] decan-4-one.

### Example 20

### 1:1 Mixture of (2R,4aR,8aR)- and (2R,4aR,8aR)-8-(4a-methyl-decahydro-naphthalen-2-yl)-1-phenyl-1,3,8-triaza-spiro[4.5]decan-4-one hydrochloride (1:1)

The title compound, m.p. > 250 °C and MS: m/e = 382 (M+H⁺) was prepared in accordance with the general method of example 1 from (4aR-trans)-octahydro-4a-methyl-2(1H)-naphthalenone and 1-phenyl-1,3,8-triazaspiro [4.5] decan-4-one.

### Example 21

### 8-(Decahydro-naphthalen-2-yl)-3-methyl-1-phenyl-1,3,8-triaza-spire[4.5]decan-4-one hydrochloride (1:1) (mixture of the diast. racemates)

8-(Decahydro-naphthalen-2-yl)-1-phenyl-1,3,8-triaza-spire[4.5]decan-4-one hydrochloride (1:1) (1.2 mmol) was suspended in DMF (20 ml). Sodium hydride (2.4 mmol) was added and the mixture was stirred at 80 °C for 1 h. The mixture was cooled to room temperature, methyl iodide (1.2 mmol) was added and the mixture was stirred for 18 h. DMF was evaporated, sodium bicarbonate and ethylacetate were added. The organic phase was washed with brine, dried with MgSO₄ and concentrated. Chromatography on silica gel (methylenchloride/methanol, 98:2) yielded the desired product which was crystallized as its HCl-salt from ethylacetate/ethanol. 0.19 g (36%) 8-(decahydro-naphthalen-2-yl)-3-methyl-1-phenyl-1,3,8-triaza-spiro[4.5]decan-4-one hydrochloride (1:1) as a colorless solid, m.p. > 250 °C and MS: m/e = 382 (M+H⁺).

### Example 22

### cis-8-(4-Methyl-cyclohexyl)-1-phenyl-1,3,8-triaza-spiro[4.5]decan-4-one hydrochloride (1:1)

The title compound, m.p. > 250 °C and NMR (DMSO-d₆): 0.96 (d, 3H), 1.9-1.5 (m, 11H), 2.99 (t, 2H), 3.16 (m, 1H), 3.43 (m, 2H), 3.66 (m, 2H), 4.62 (s, 2H), 6.79 (t, 1H), 7.08 (d, 2H), 7.22 (t, 2H), 9.02 (s, 1H), 10.35 (bs, 1H) was isolated by preparative HPLC of the mixture obtained in example 4.

### Example 23

### trans-8-(4-Methyl-cyclohexyl)-1-phenyl-1,3,8-triaza-spiro[4.5]decan-4-one hydrochloride (1:1)

The title compound, m.p. > 250 °C and NMR (DMSO-d₆): 0.87 (d, 3H), 0.97 (t, 2H), 1.3 (m, 1H), 1.54 (m, 2H), 1.90 (t, 2H), 2.08 (d, 2H), 2.97 (t, 2H), 3.16 (m, 1H), 3.4 (m, 2H), 3.70 (m, 2H), 4.62 (s, 2H), 6.81 (t, 1H), 7.08 (d, 2H), 7.22 (t, 2H), 9.02 (s, 1H), 10.35 (bs, 1H) was isolated by preparative HPLC of the mixture obtained in example 4.

### Example 24

### cis-8-[4-(1,1-Dimethyl-propyl)-cyclohexyl]-1-phenyl-1,3,8-triaza-spiro[4.5]decan-4-one hydrochloride (1:1)

The title compound, m.p. > 250 °C and MS: m/e = 384 (M+H⁺) was prepared in accordance with the general method of example 1 from 4-(1,1-dimethylpropyl)-cyclohexanone and 1-phenyl-1,3,8-triazaspiro[4.5]decan-4-one.

### Example 25

### trans-8-[4-(1,1-Dimethyl-propyl)-cyclohexyl]-1-phenyl-1,3,8-triaza-spiro[4.5]decan-4-one hydrochloride (1:1)

The title compound, m.p. > 250 °C and MS: m/e = 384 (M+H⁺) was prepared in accordance with the general method of example 1 from 4-(1,1-dimethylpropyl)-cyclohexanone and 1-phenyl-1,3,8-triazaspiro[4.5]decan-4-one.

### Example 26

### Mixture of (2RS,4aRS,8aSR)- and (2SR,4aRS,8aSR)-8-(decahydro-naphthalen-2-yl)-1-phenyl-1,3,8-triaza-spiro[4.5]decan-4-one hydrochloride (1:1)

The title compound, m.p. > 250 °C and MS: m/e = 368 (M+H⁺) was prepared in accordance with the general method of example 1 from cis-octahydro-2(1H)-naphthalenone and 1-phenyl-1,3,8-triazaspiro[4.5]decan-4-one.

### Example 27

### Mixture of cis- and trans 8-(4-Cyclohexyl-cyclohexyl)-1-phenyl-1,3,8-triaza-spiro[4.5]decan-4-one hydrochloride (1:1)

The title compound, m.p. > 250 °C and MS: m/e = 356.3 (M+H⁺) was prepared in accordance with the general method of example 1 from 4-cydohexylcydohexanone and 1-phenyl-1,3,8-triazaspiro[4.5]decan-4-one.

### Example 28

### Mixture of cis- and trans 8-(4-Isopropyl-cyclohexyl)-1-phenyl-1,3,8-triaza-spiro[4.5]decan-4-one hydrochloride (1:1)

The title compound, m.p. > 250 °C and MS: m/e = 384 (M+H⁺) was prepared in accordance with the general method of example 1 from 4-isopropylcyclohexanone and 1-phenyl-1,3,8-triazaspiro [4.5]decan-4-one.

### Example 29

### 8-Cyclododecyl-1-phenyl-1,3,8-triaza-spiro[4,5]decan-4-one hydrochloride (1:1)

The title compound, white solid, m.p. > 230 °C and MS: m/e = 398.4 (M+H⁺) was prepared in accordance with the general method of example 1 from cydododecanone and 1-phenyl-1,3,8-triazaspiro[4,5]decan-4-one.

### Example 30

### 8-[2-(2-norbornyl)-cyclopentyl]-1-phenyl-1,3,8-triaza-spiro[4,5]decan-4-one hydrochloride (1:1) (mixture of diastereoisomers)

The tide compound, white solid, m.p. > 230 °C and MS: m/e = 394.3 (M+H⁺) was prepared in accordance with the general method of example 7 from rac-2-(2-norbornyl)-cyclopentanone and 1-phenyl-1,3,8-triazaspiro[4,5]decan-4-one.

### Example 31

### 8-Cyclodecyl-1-phenyl-1,3,8-triaza-spiro[4,5]decan-4-one hydrochloride (1:1)

The title compound, white solid, m.p. > 220 °C and MS: m/e = 370.3 (M+H⁺) was prepared in accordance with the general method of example 1 from cyclodecanone and 1-phenyl-1,3,8-triazaspiro[4,5]decan-4-one.

### Example 32

### 8-Cycloheptyl-1-phenyl-1,3,8-triaza-spiro[4,5]decan-4-one hydrochloride (1:1)

The title compound, white solid, m.p. > 230 °C and MS: m/e = 328.3 (M+H⁺) was prepared in accordance with the general method of example 1 from cycloheptanone and 1-phenyl-1,3,8-triazaspiro[4,5]decan-4-one.

### Example 33

### 8-Cyclooctyl-1-phenyl-1,3,8-triaza-spiro[4,5]decan-4-one hydrochloride (1:1)

The tide compound, white solid, m.p. > 230 °C and MS: m/e = 328.3 (M+H⁺) was prepared in accordance with the general method of example 1 from cyclooctanone and 1-phenyl-1,3,8-triazaspiro [4,5] decan-4-one.

### Example 34

### 8-Cyclononyl-1-phenyl-1,3,8-triaza-spiro[4,5]decan-4-one hydrochloride (1:1)

The title compound, white solid, m.p. > 220 °C and MS: m/e = 356.3 (M+H⁺) was prepared in accordance with the general method of example 1 from cyclononanone and 1-phenyl-1,3,8-triazaspiro[4,5]decan-4-one.

### Example 35

### 8-Cycloundecyl-1-phenyl-1,3,8-triaza-spiro[4,5]decan-4-one hydrochloride (1:1)

The title compound, white solid, m.p. > 220 °C and MS: m/e = 384.3 (M+H⁺) was prepared in accordance with the general method of example 1 from cycloundecanone and 1-phenyl-1,3,8-triazaspiro [4,5]decan-4-one.

### Example 36

### 8-(cis-2,3,3a,4,5,9b-Hexahydro-1H-cyclopenta[a]naphthalen-2-yl)-1-phenyl-1,3,8-triaza-spiro[4,5]decan-4-one hydrochloride (1:1) (mixture of diastereoisomers)

The title compound, white solid, m.p. 217 °C (dec.) and MS: m/e = 402.4 (M+H⁺) was prepared in accordance with the general method of example 1 from cis-1,3,3a,4,5,9b-hexahydro-1H-cyclopenta[a] naphthalen-2-one and 1-phenyl-1,3,8-triazaspiro[4,5]decan-4-one.

### Example 37

### 8-(2,3,4,5-Tetrahydro-cyclopenta[a]naphthalen-2-yl)-1-phenyl-1,3,8-triaza-spiro[4,5]decan-4-one hydrochloride (1:1)

The title compound, white solid, m.p. > 250 °C and MS: m/e = 400.4 (M+H⁺) was prepared in accordance with the general method of example 1 from 1,3,4,5-tetrahydrocyclopenta[a]naphthalen-2-one and 1-phenyl-1,3,8-triazaspiro[4,5]decan-4-one.

### Example 38

### 8-(cis-Octahydro-inden-2-yl)-1-phenyl-1,3,8-triaza-spiro[4,5]decan-4-one hydrochloride (1:1)

The title compound, white solid, m.p. 271 °C (dec.) and MS: m/e = 354.4 (M+H⁺) was prepared in accordance with the general method of example 1 from cis-octahydro-inden-2-one and 1-phenyl-1,3,8-triazaspiro[4,5]decan-4-one.

### Example 39

### 8- (4a,10a-cis-1,2,3,4,4a,9,10,10a-Octahydro-phenanthren-3-yl)-1-phenyl-1,3,8-triaza-spiro[4,5]decan-4-one hydrochloride (1:1) (mixture of diastereoisomers)

The title compound, white solid, m.p. 257 °C and MS: m/e = 416.2 (M+H⁺) was prepared in accordance with the general method of example 1 from (RS)-cis-1,4,4a,9,10,10a-hexahydro-2H-phenanthren-3-one and 1-phenyl-1,3,8-triazaspiro[4,5] decan-4-one.

### Example 40

### 8-(cis-Octahydro-inden-1-yl)-1-phenyl-1,3,8-triaza-spire[4,5]decan-4-one hydrochloride (1:1) (mixture of diastereoisomers)

The title compound, white solid, m.p. 242 °C and MS: m/e = 354.3 (M+H⁺) was prepared in accordance with the general method of example 1 from (RS)-cis-octahydro-inden-1-one and 1-phenyl-1,3,8-triazaspiro[4,5]decan-4-one.

### Example 41

### 8-(cis-Decahydro-azulen-1-yl)-1-phenyl-1,3,8-triaza-spiro[4,5]decan-4-one hydrochloride (1:1) (mixture of diastercoisomers)

The title compound, white solid, m.p. 259 °C and MS: m/e = 368.3 (M+H⁺) was prepared in accordance with the general method of example 1 from (RS)-cis-octahydro-azulen-1-one and 1-phenyl-1,3,8-triazaspiro[4,5]decan-4-one.

### Example 42

### 8-Cyclodecyl-3-methyl-1-phenyl-1,3,8-triaza-spiro[4,5]decan-4-one hydrochloride (1:1)

The title compound, white solid, m.p. 248 °C (dec.) and MS: m/e = 384.3 (M+H⁺) was prepared in accordance with the general method of example 21 from 8-cyclodecyl-1-phenyl-1,3,8-triaza-spiro[4,5]decan-4-one hydrochloride and methyliodide.

### Example 43

### 8-Cyclodecyl-3-ethyl-1-phenyl-1,3,8-triaza-spiro[4,5]decan-4-one hydrochloride (1:1)

The title compound, white solid, m.p. 197 °C (dec.) and MS: m/e = 398.4 (M+H⁺) was prepared in accordance with the general method of example 21 from 8-cyclodecyl-1-phenyl-1,3,8-triaza-spiro [4,5] decan-4-one hydrochloride and ethyliodide.

### Example 44

### 8-Cyclodecyl-3-benzyl-1-phenyl-1,3,8-triaza-spiro[4,5]decan-4-one hydrochloride (1:1)

The title compound, white solid, m.p. 217 °C (dec.) and MS: m/e = 460.4 (M+H⁺) was prepared in accordance with the general method of example 21 from 8-cyclodecyl-1-phenyl-1,3,8-triaza-spiro[4,5]decan-4-one hydrochloride and benzylbromide.

### Example 45

### 8-Cyclodecyl-3-(3-phenyl-1-propyl)-1-phenyl-1,3,8-triaza-spiro[4,5]decan-4-one hydrochloride (1:1)

The title compound, white solid, m.p. 164 °C and MS: m/e = 488.4 (M+H⁺) was prepared in accordance with the general method of example 21 from 8-cyclodecyl-1-phenyl-1,3,8-triaza-spiro[4,5]decan-4-one hydrochloride and 3-phenyl-1-propylbromide.

### Example 46

### 8-Cyclodecyl-3-(3,3-diphenyl-1-propyl)-1-phenyl-1,3,8-triaza-spiro[4,5]decan-4-one hydrochloride (1:1)

The title compound, white solid, m.p. 219 °C and MS: m/e = 564.5 (M+H⁺) was prepared in accordance with the general method of example 21 from 8-cyclodecyl-1-phenyl-1,3,8-triaza-spiro[4,5]decan-4-one hydrochloride and 3,3-diphenyl-1-propylbromide.

### Example 47

### 8-(1-Isopropyl-4-methyl-bicyclo[3.1.0]hex-3-yl)-1-phenyl-1,3,8-triaza-spire[4,5]decan-4-one hydrochloride (1:1) (mixture of diastereoisomers)

The title compound, white solid, m.p. 275 °C and MS: m/e = 368.3 (M+H⁺) was prepared in accordance with the general method of example 7 from a/b-thujone and 1-phenyl-1,3,8-triazaspiro[4,5]decan-4-one.

### Example 48

### (RS)-8-[Spiro(5,5)undecan-2-yl]-1-phenyl-1,3,8-triaza-spiro[4,5]decan-4-one hydrochloride (1:1)

The title compound, white solid, m.p. 289 °C (dec.) and MS: m/e = 381 (M⁺) was prepared in accordance with the general method of example 7 from spiro(5,5)undecan-2-one and 1-phenyl-1,3,8-triazaspiro[4,5]decan-4-one.

### Example 49

### 8-(Decahydro-azulen-2-yl)-1-phenyl-1,3,8-triaza-spiro[4,5]decan-4-one hydrochloride (1:1) (mixture of diastereoisomers)

The title compound, white solid, m.p. 272 °C and MS: m/e = 368.3 (M+H⁺) was prepared in accordance with the general method of example 1 from (RS)-octahydro-azulen-2-one and 1-phenyl-1,3,8-triazaspiro[4,5]decan-4-one.

### Example 50

### Mixture of cis- and trans 1-Phenyl-8-(4-trifluoromethyl-cyclohexyl)-1,3,8-triaza-spiro[4.5]decan-4-one hydrochloride (1:1)

The title compound, m.p. > 250 °C and MS: m/e = 382.2 (M+H⁺) was prepared in accordance with the general method of example 1 from 4-trifluoromethylcyclohexanone and 1-phenyl-1,3,8-triazaspiro[4.5]decan-4-one.

### Example 51

### cis-8-(4-Isopropyl-cyclohexyl)-1-phenyl-1,3,8-triaza-spiro[4.5]decan-4-one hydrochloride (1:1)

The title compound, m.p. > 250 °C and NMR (DMSO-d₆): 0.88 (d, 6H), 2.0-1.1 (m, 12H), 2.97 (t, 2H), 3.25 (m, 1H), 3.47 (m, 2H), 3.67 (m, 2H), 4.62 (s, 2H), 6.79 (t, 1H), 7.08 (d, 2H), 7.22 (t, 2H), 9.02 (s, 1H), 10.30 (bs, 1H) was isolated by preparative HPLC of the mixture obtained in example 28.

### Example 52

### trans-8-(4-Isopropyl -cyclohexyl)-1-phenyl-1,3,8-triaza-spiro[4.5]decan-4-one hydrochloride (1:1)

The title compound, m.p. > 250 °C and NMR (DMSO-d₆): 0.85 (d, 6H), 1.05 (m, 3H), 1.48 (m, 3H), 1.83 (m, 4H), 2.13 (m, 2H), 2.97 (t, 2H), 3.16 (m, 1H), 3.4 (m, 2H), 3.70 (m, 2H), 4.62 (s, 2H), 6.79 (t, 1H), 7.08 (d, 2H), 7.22 (t, 2H), 9.02 (s, 1H), 10.50 (bs, 1H) was isolated by preparative HPLC of the mixture obtained in example 28.

### Example 53

### (R,S)-8-Cyclodecyl-1-phenyl-2-methyl-1,3,8-triaza-spiro[4,5]decan-4-one hydrochloride (1:1)

8-Benzyl-1-phenyl-1,3,8,-triaza-spiro[4,5]dec-2-en-4-one (1 g, 3.2 mMol) and some crystals of Cu(I)-chloride was dissolved in 50 ml dry THF and methyl-magnesiumchloride (2.1 ml of a 3M solution in THF) was added dropwise. The reaction was stirred at RT until the TLC showed the absence of starting-material. To the reaction mixture was added 50 ml of a saturated ammoniumchloride solution and stirred for 30 minutes. Then the layers were separated and the aqueous layer was extracted three times with ethyl-acetate.The combined organic layers were dried over sodium sulfate, filtered and evaporated to give 1.05 g of (R,S)-8-benzyl-2-methyl-1-phenyl-1,3,8,-triaza-spiro[4,5]decan-4-one as slight yellow glass, m.p. 64.3-65.8 °C, suitable clean for the next step.

1.05g of this compound was dissolved in 30 ml methanol, 300 mg of Pd/C 10% was added and a balloon with hydrogen was attached. When TLC indicated the absence of starting material, the reaction mixture was filtered and evaporated to yield 617 mg of 2-methyl-1-phenyl-1,3,8,-triaza-spiro[4,5]decan-4-on as a slightly yellow foam. MS: 246 ((M+H)⁺), suitable clean for the next reaction.

617 mg of this compound were dissolved in THF and 2.2 ml tetraisopropylortho-titanate and 2.8 mmol cyclodecanon was added. After 18 h at roomtemperature, the mixture was evaporated and the residue dissolved in ethanol and 2.5 mMol of sodiumcyanoborohydride was added. After 18 h at room-temperature, water and ethyl acetate was added, the layers separated and the organic layer washed with 2N aqueous sodium hydroxide solution and brine. The organic layer was dried over sodium sulfate, filtered and evaporated. Chromatography on silica-gel (ethyl-acetate) yielded the desired product which was crystallized as its hydrochloride salt from methanol/ether. 41 mg of (R,S)-8-Cyclodecyl-1-phenyl-2-methyl-1,3,8-triaza-spiro[4,5]decan-4-one hydrochloride (1:1) as an off-white powder, m.p.>250 °C, MS: m/e = 384 ((M+H)⁺).

### Example 54

### (R,S)-8-Cyclodecyl-1,2-diphenyl-1,3,8-triaza-spiro[4,5]decan-4-one hydrochloride (1:1)

The title compound, m.p. > 250 °C, MS: m/e = 446 ((M+H)⁺), was prepared in accordance with the general method of example 53 from 8-benzyl-1-phenyl-13,8,-triaza-spiro[4,5]dec-2-en-4-on, phenyl-magnesium chloride and cyclodecanone.

### Example 55

### (R,S)-8-Cyclodecyl-1-(4-methyl-phenyl)-2-butyl-1,3,8-triaza-spiro[4,5]decan-4-one hydrochloride (1:1)

The title compound, white crystals, MS: m/e = 440 ((M+H)⁺), was prepared in accordance with the general method of example 53 from 8-benzyl-1-(4-methyl-phenyl)-1,3,8,-triaza-spiro[4,5]dec-2-en-4-on, butyl-magnesium chloride and cyclodecanone.

### Example 56

### (R,S)-8-Cyclodecyl-1-(2-methyl-phenyl)-2-butyl-1,3,8-triaza-spiro[4,5]decan-4-one hydrochloride (1:1)

The title compound, yellow powder, MS: m/e = 440 ((M+H)⁺), was prepared in accordance with the general method of example 53 from 8-benzyl-1-(2-methyl-phenyl)-1,3,8,-triaza-spire[4,5]dec-2-en-4-on, butyl-magnesium chloride and cyclodecanone.

### Example 57

### (R,S)-8-Cyclodecyl-1-(3-methyl-phenyl)-2-phenyl-1,3,8-triaza-spiro[4,5]decan-4-one hydrochloride (1:1)

The title compound, white crystals, MS: m/e = 460 ((M+H)⁺), was prepared in accordance with the general method of example 53 from 8-benzyl-1-(3-methyl-phenyl)-1,3,8,-triaza-spiro[4,5]dec-2-en-4-on, phenyl-magnesium chloride and cyclodecanone.

### Example 58

### (R,S)-8-Cyclodecyl-1-(3-methyl-phenyl)-2-methyl-1,3,8-triaza-spiro[4,5]decan-4-one hydrochloride (1:1)

The title compound, white crystals, m.p. >250 °C, MS: m/e = 398 ((M+H)⁺), was prepared in accordance with the general method of example 53 from 8-benzyl-1-(3-methyl-phenyl)-1,3,8,-triaza-spire[4,5]dec-2-en-4-on, methyl-magnesium chloride and cyclodecanone.

### Example 59

### (R,S)-8-Cyclodecyl-1-(3-methyl-phenyl)-2-butyl-1,3,8-triaza-spiro[4,5]decan-4-one hydrochloride (1:1)

The title compound, off-white crystals, m.p. >250 °C, MS: m/e = 440 ((M+H)⁺), was prepared in accordance with the general method of example 53 from 8-benzyl-1-(3-methyl-phenyl)-1,3,8,-triaza-spiro[4,5]dec-2-en-4-on, butyl-magnesium chloride and cyclodecanone.

### Example 60

### 8-Cyclodecyl-1-(2-methyl-phenyl)-1,3,8-triaza-spiro[4,5]decan-4-one hydrochloride (1:1)

The tide compound, yellow crystals, MS: m/e = 384 ((M+H)⁺), was prepared in accordance with the general method of example 1 from 1-(2-methyl-phenyl)-1,3,8,-triaza-spiro[4,5]decan-4-on and cyclodecanone.

### Example 61

### 8-Cyclodecyl-1-(4-methyl-phenyl)-1,3,8-triaza-spiro[4,5]decan-4-one hydrochloride (1:1)

The title compound, yellow crystals, MS: m/e = 384 ((M+H)⁺), was prepared in accordance with the general method of example 1 from 1-(4-methyl-phenyl)-1,3,8,-triaza-spiro[4,5]decan-4-on and cyclodecanone.

### Example 62

### 8-(Dodecahydro-acenaphthylen-1-yl)-1-phenyl-1,3,8-triaza-spiro[4,5]decan-4-one hydrochloride (1:1) (mixture of diastereoisomers)

The title compound, white solid, m.p. 265 °C and MS: m/e = 394.3 (M+H⁺) was prepared in accordance with the general method of example 7 from rac-dodecahydroacenaphthylen-1-one and 1-phenyl-1,3,8-triazaspiro[4,5]decan-4-one.

### Example 63

### 8-(cis-Bicyclo[6.2.0]dec-9-yl)-1-phenyl-1,3,8-triaza-spiro[4,5]decan-4-one hydrochloride (1:1) (mixture of diastereoisomers)

The title compound, white solid, m.p. 242 °C and MS: m/e = 368.3 (M+H⁺) was prepared in accordance with the general method of example 7 from (RS)-cis-bicyclo[6.2.0]dec-9-one and 1-phenyl-1,3,8-triazaspiro [4,5] decan-4-one.

### Example 64

### cis-8-(4-Cyclohexyl-cyclohexyl)-1-phenyl-1,3,8-triaza-spiro[4.5]decan-4-one hydrochloride (1:1)

The title compound, m.p. >250 °C and NMR (DMSO-d₆): 0.80 (m, 2H), 2.0-1.1 (m, 20H), 2.97 (t, 2H), 3.25 (m, 1H), 3.47 (m, 2H), 3.70 (m, 2H), 4.62 (s, 2H), 6.80 (t, 1H), 7.05 (d, 2H), 7.22 (t, 2H), 9.02 (s, 1H), 10.0 (bs, 1H) was isolated by preparative HPLC of the mixture obtained in example 27.

### Example 65

### trans-8-(4-Propyl-cyclohexyl)-1-phenyl-1,3,8-triaza-spiro[4.5]decan-4-one hydrochloride (1:1)

The title compound, m.p. >250 °C and NMR (DMSO-d₆): 0.86 (t, 3H), 1.6-0.9 (m, 10H), 1.85 (d, 4H), 2.15 (d, 2H), 3.0 (t, 2H), 3.20 (m, 1H), 3.43 (m, 2H), 3.70 (m, 2H), 4.62 (s, 2H), 6.78 (t, 1H), 7.05 (d, 2H), 7.18 (t, 2H), 9.02 (s, 1H), 10.55 (bs, 1H) was isolated by preparative HPLC of the mixture obtained in example 13.

### Example 66

### cis-8-(4-Propyl-cyclohexyl)-1-phenyl-1,3,8-triaza-spiro[4.5]decan-4-one hydrochloride (1:1)

The title compound, m.p. >250 °C and NMR (DMSO-d₆): 0.89 (t, 3H), 2.0-1.1 (m, 15H), 2.95 (t, 2H), 3.15 (m, 1H), 3.45 (m, 2H), 3.70 (m, 2H), 4.62 (s, 2H), 6.78 (t, 1H), 7.08 (d, 2H), 7.22 (t, 2H), 9.02 (s, 1H), 10.40 (bs, 1H) was isolated by preparative HPLC of the mixture obtained in example 13.

### Example 67

### (2RS,4aRS,8aSR)-8-(decahydro-naphthalen-2-yl)-1-phenyl-1,3,8-triaza-spiro[4.5]decan-4-one hydrochloride (1:1)

1-(2RS,4aRS,8aSR-Decahydro-naphthalen-2-yl)-4-phenylamino-piperidine-4-carboxylic acid amide (8 mmol) suspended in formamide (40 ml) was stirred for 2 h at 200 °C. The mixture was cooled, poured into cold water (400 ml) and extracted with methylene chloride. Organic phases were pooled, dried with sodium sulfate and concentrated to yield a mixture of (2RS,4aRS,8aSR)-8-(decahydro-naphthalen-2-yl)-1-phenyl-1,3,8-triaza-spiro[4.5]decan-4-one and (2RS,4aRS,8aSR)-8-(decahydro-naphthalen-2-yl)-1-phenyl-1,3,8-triaza-spiro[4.5]dec-2-en-4-one. This mixture was dissolved in methanol (60 ml) and sodiumborohydride (12 mmol) was added. The mixture was stirred for 1 h at 60 °C, cooled and concentrated. Saturated ammonium chloride solution and methylene chloride were added to the residue. The water phase was extracted with methylene chloride. Organic phases were pooled, dried with sodium sulfate and concentrated. Chromatography on silica gel (methylene chloride/methanol, 98:2) yielded the desired product. This was crystallized as its HCl-salt from ethylacetate/ethanol. 0.65 g (22%) of (2RS,4aRS,8aSR)-8-(decahydro-naphthalen-2-yl)-1-phenyl-1,3,8-triaza-spiro[4.5]decan-4-one hydrochloride (1:1), m.p. >250 °C and MS: m/e = 368.2 (M+H⁺).

### Example 68

### 8-Cyclodecyl-3-(3-methoxy-benzyl)-1-phenyl-1,3,8-triaza-spiro[4.5]decan-4-one hydrochloride (1:1)

The title compound, white solid, m. p. 175°C and MS: m/e = 490.4 (M+H⁺) was prepared in accordance with the general method of example 21 from 8-cyclodecyl-1-phenyl-1,3,8-triaza-spiro[4.5]decan-4-one hydrochloride and 3-methoxy-benzylchloride.

### Example 69

### 8-Cyclodecyl-3-(4,6-dimethoxy-[1,3,5]triazin-2-yl)-1-phenyl-1,3,8-triaza-spiro[4.5]decan-4-one hydrochloride (1:1)

The tide compound, white solid, m. p. 216°C and MS: m/e = 509.5 (M+H⁺) was prepared in accordance with the general method of example 21 from 8-cyclodecyl-1-phenyl-1,3,8-triaza-spiro[4.5]decan-4-one hydrochloride and 2-chloro-4,6-dimethoxy- [1,3,5]triazine.

### Example 70

### 8-Cyclodecyl-1-phenyl-3-(4-phenyl-butyl)-1,3,8-triaza-spiro[4.5]decan-4-one hydrochloride (1:1)

The title compound, white solid, m. p. 115°C and MS: m/e = 502.5 (M+H⁺) was prepared in accordance with the general method of example 21 from 8-cyclodecyl-1-phenyl-1,3,8-triaza-spiro[4.5]decan-4-one hydrochloride and methanesulfonic acid 4-phenyl-but-1-yl ester.

### Example 71

### (8-Cyclodecyl-4-oxo-1-phenyl-1,3,8-triaza-spiro[4.5]dec-3-yl)-acetonitrile hydrochloride (1:1)

The title compound, white solid, m. p. 240°C and MS: m/e = 409.3 (M+H⁺) was prepared in accordance with the general method of example 21 from 8-cyclodecyl-1-phenyl-1,3,8-triaza-spiro[4.5]decan-4-one hydrochloride and bromoacetonitrile.

### Example 72

### 8-Cyclodecyl-1-phenyl-3- (2-piperidin-1-yl-ethyl)-1,3,8-triaza-spiro[4.5]decan-4-one hydrochloride (1:2)

The title compound, white solid, m. p. 245°C and MS: mle = 481.5 (M+H⁺) was prepared in accordance with the general method of example 21 from 8-cyclodecyl-1-phenyl-1,3,8-triaza-spiro[4.5]decan-4-one hydrochloride and 1-(2-chloroethyl)-piperidine.

### Example 73

### 8-Cyclodecyl-3-naphthalen-1-ylmethyl-1-phenyl-1,3,8-triaza-spiro[4.5]decan-4-one hydrochloride (1:1)

The title compound, white solid, m. p. 210°C and MS: m/e = 510.4 (M+H⁺) was prepared in accordance with the general method of example 21 from 8-cyclodecyl-1-phenyl-1,3,8-triaza-spiro[4.5]decan-4-one hydrochloride and 1-chloromethyl-naphthaline.

### Example 74

### 8-Cyclodecyl-3-cyclopentyl-1-phenyl-1,3,8-triaza-spiro[4.5]decan-4-one hydrochloride (1:1)

The title compound, white solid, m. p. 221°C and MS: m/e = 438.3 (M+H⁺) was prepared in accordance with the general method of example 21 from 8-cydodecyl-1-phenyl-1,3,8-triaza-spiro[4.5]decan-4-one hydrochloride and bromocyclopentane.

### Example 75

### 8-Cyclodecyl-1-phenyl-3-(3-trifluoromethyl-benzyl)-1,3,8-triaza-spiro[4.5]decan-4-one hydrochloride (1:1)

The title compound, white solid, m. p. 197°C and MS: m/e = 528.3 (M+H⁺) was prepared in accordance with the general method of example 21 from 8-cyclodecyl-1-phenyl-1,3,8-triaza-spiro[4.5]decan-4-one hydrochloride and 3-trifluoromethyl-benzylchloride.

### Example 76

### 8-Cyclodecyl-1-phenyl-3-(3-piperidin-1-yl-propyl)-1,3,8-triaza-spiro[4.5]decan-4-one hydrochloride (1:2)

The title compound, white solid, m. p. 180°C and MS: m/e = 495.3 (M+H⁺) was prepared in accordance with the general method of example 21 from 8-cyclodecyl-1-phenyl-1,3,8-triaza-spiro[4.5]decan-4-one hydrochloride and methanesulfonic acid 3-(1-piperidino)-prop-1-yl ester.

### Example 77

### 8-Cyclodecyl-3-cyclopropylmethyl-1-phenyl-1,3,8-triaza-spiro[4.5]decan-4-one hydrochloride (1:1)

The title compound, white solid, m. p. 214°C and MS: m/e = 424.3 (M+H⁺) was prepared in accordance with the general method of example 21 from 8-cyclodecyl-1-phenyl-1,3,8-triaza-spiro[4.5]decan-4-one hydrochloride and bromomethyl-cyclopropane.

### Example 78

### 8-Cyclodecyl-1-phenyl-3-pyridin-3-ylmethyl-1,3,8-triaza-spiro[4.5]decan-4-one hydrochloride (1:2)

The title compound, white solid, m. p. 227°C and MS: m/e = 461.3 (M+H⁺) was prepared in accordance with the general method of example 21 from 8-cyclodecyl-1-phenyl-1,3,8-triaza-spiro[4.5]decan-4-one hydrochloride and 3-chloromethyl-pyridine.

### Example 79

### 8-Cyclodecyl-3-(2-morpholin-4-yl-ethyl)-1-phenyl-1,3,8-triaza-spiro[4.5]decan-4-one hydrochloride (1:2)

The title compound, white solid, m. p. 190°C and MS: m/e = 483.6 (M+H⁺) was prepared in accordance with the general method of example 21 from 8-cyclodecyl-1-phenyl-1,3,8-triaza-spiro[4.5]decan-4-one hydrochloride and N-(2-chloroethyl)-morpholine.

### Example 80

### 8-Cyclodecyl-3-(2-[1.3]dioxolan-2-yl-ethyl)-1-phenyl-1,3,8-triaza-spiro[4.5]decan-4-one hydrochloride (1:1)

The title compound, white solid, m. p. 205°C and MS: m/e = 470.6 (M+H⁺) was prepared in accordance with the general method of example 21 from 8-cydodecyl-1-phenyl-1,3,8-triaza-spire[4.5]decan-4-one hydrochloride and 2-(2-bromoethyl)-1,3-dioxolane.

### Example 81

### 8-Cyclodecyl-3-[2-(5-methyl-2-phenyl-oxazol-4-yl)-ethyl]-1-phenyl-1,3,8-triaza-spiro[4.5]decan-4-one hydrochloride (1:1)

The title compound, white solid, m. p. 140°C and MS: m/e = 555.3 (M+H⁺) was prepared in accordance with the general method of example 21 from 8-cyclodecyl-1-phenyl-1,3,8-triaza-spiro[4.5]decan-4-one hydrochloride and methanesulfonic acid 2-(5-methyl-2-phenyloxazol-4-yl)-ethyl ester.

### Example 82

### 8-Cyclodecyl-1-phenyl-3- (2-pyrrolidin-1-yl-ethyl)-1,3,8-triaza-spiro[4.5]decan-4-one hydrochloride (1:2)

The title compound, white solid, m. p. 179°C and MS: m/e = 467.3 (M+H⁺) was prepared in accordance with the general method of example 21 from 8-cydodecyl-1-phenyl-1,3,8-triaza-spiro[4.5]decan-4-one hydrochloride and 1-(2-chloroethyl)-pyrrolidine.

### Example 83

### 8-Cyclodecyl-3-(2-oxo-oxazolidin-5-ylmethyl)-1-phenyl-1,3,8-triaza-spiro[4.5]decan-4-one hydrochloride (1:1)

The title compound, white solid, m. p. 174°C and MS: m/e = 469.3 (M+H⁺) was prepared in accordance with the general method of example 21 from 8-cyclodecyl-1-phenyl-1,3,8-triaza-spiro[4.5]decan-4-one hydrochloride and 5-chloromethyl-2-oxazolidinone.

### Example 84

### (R,S)-8-Cyclodecyl-1-(4-methyl-phenyl)-2-methyl-1,3,8-triaza-spiro[4,5]decan-4-one hydrochloride (1:1)

The title compound, white crystals, MS: m/e = 398 ((M+H)⁺), was prepared in accordance with the general method of example 53 from 8-benzyl-1-(4-methyl-phenyl)-1,3,8,-triaza-spiro[4,5]dec-2-en-4-on, methyl-magnesium chloride and cyclodecanone

### Example 85

### (8-Cyclodecyl-4-oxo-1-phenyl-1,3,8-triaza-spiro[4,5]dec-3-yl)-acetic acid methyl ester hydrochloride (1:1)

The title compound, white solid, m. p. 178°C and MS: m/e = 442.5 (M+H⁺) was prepared in accordance with the general method of example 21 from 8-cydodecyl-1-phenyl-1,3,8-triaza-spiro[4.5]decan-4-one hydrochloride and bromomethyl acetate.

### Example 86

### 2-(8-Cyclodecyl-4-oxo-1-phenyl-1,3,8-triaza-spiro[4.5]dec-3-yl)-acetamide hydrochloride (1:1)

(8-Cyclodecyl-4-oxo-1-phenyl-1,3,8-triaza-spiro[4.5]dec-3-yl)-acetic acid methyl ester (0.7 mmol) was dissolved in a 9M solution of ammonia in methanol (5 ml). The reaction mixture was shaken overnight in a closed tube at 50°C. After evaporation of the solvent the desired product remained as a white foam. Formation of the HCl-salt yielded 0.255 g of the title compound as a white solid, m. p. >250°C and MS: m/e = 427.5 (M+H⁺).

### Example 87

### 2-(8-Cyclodecyl-4-oxo-1-phenyl-1,3,8-triaza-spiro[4.5]dec-3-yl)-N,N-dimethylacetamide hydrochloride (1:1)

The title compound, white solid, m. p. 145°C and MS: m/e = 455.6 (M+H⁺) was prepared in accordance with the general method of example 21 from 8-cyclodecyl-1-phenyl-1,3,8-triaza-spiro[4.5]decan-4-one hydrochloride and N,N-dimethyl-2-chloroacetamide.

### Example 88

### 8-Cyclodecyl-3-(2-hydroxy-ethyl)-1-phenyl-1,3,8-triaza-spiro[4.5]decan-4-one hydrochloride (1:1)

(8-Cyclodecyl-4-oxo-1-phenyl-1,3,8-triaza-spiro[4.5]dec-3-yl)-acetic acid methyl ester (0.8 mmol) was dissolved in ethyleneglycol dimethylether (2ml). Lithiumchloride (0.8 mmol) and sodium borohydride (0.8 mol) were added. After stirring for 4 h the reaction mixture was distributed between dichloromethane/water/methanol. The organic layer was separated, dried over magnesium sulfate and evaporated. The residue was purified by column chromatography (SiO2, ethyl acetate). Formation of the HCl-salt yielded the title compound as a white solid, 0.124 g, m. p. 190°C and MS: m/e = 414.5 (M+H⁺).

### Example 89

### N-Benzyl-2-(8-cyclodecyl-4-oxo-1-phenyl-1,3,8-triaza-spiro[4.5]dec-3-yl)-acetamide hydrochloride (1:1)

(8-Cyclodecyl-4-oxo-1-phenyl-1,3,8-triaza-spiro[4.5]dec-3-yl)-acetic acid methyl ester (0.6 mmol) was dissolved in methanol (5 ml). After adding benzylamine (1.2 mmol) the reaction mixture was kept 4 days at room temperature. The solvent was evaporated and the residue was purified by column chromatography (SiO2, ethyl acetate). Formation of the HCl-salt yielded 0.255 g of the title compound as a white solid, 66 mg, m. p. 196°C and MS: m/e = 517.4 (M+H⁺).

### Example 90

### 5-(8-Cyclodecyl-4-oxo-1-phenyl-1,3,8-triaza-spiro[4.5]dec-3-yl)-pentanoic acid ethyl ester hydrochloride (1:1)

The title compound, white solid, m. p. 170°C and MS: m/e = 498.5 (M+H⁺) was prepared in accordance with the general method of example 21 from 8-cyclodecyl-1-phenyl-1,3,8-triaza-spiro[4.5]decan-4-one hydrochloride and ethyl 5-bromovaleriate.

### Example 91

### 1-Phenyl-8-(trans-4-trifluoromethyl-cyclohexyl)-1,3,8-triaza-spiro[4.5]decan-4-one hydrochloride (1:1)

The title compound, m.p. >250°C and NMR (DMSO-d₆): 1.39 (q, 2H), 1.62 (q, 2H), 1.88 (d, 2H), 2.01 (d, 2H), 2.24 (d, 2H), 2.33 (bs, 1H), 2.99 (t, 2H), 3.36 (m, 3H), 3.70 (m, 2H), 4.62 (s, 2H), 6.78 (t, 1H), 7.09 (d, 2H), 7.21 (t, 2H), 9.02 (s, 1H), was isolated by preparative LC of the mixture obtained in example 50.

### Example 92

### Mixture of cis- and trans 1-Phenyl-8-(4-ethyl-cyclohexyl)-1,3,8-triaza-spiro[4.5]decan-4-one hydrochloride (1:1)

The title compound, m.p. >250°C and NMR (DMSO-d₆): 0.86 (t, 3H), 0.9- 2.0 (m, 12H), 2.20 (d, 1H), 3.1 (m, 3H), 3.4 (m, 2H), 3.70 (m, 2H), 4.62 (s, 2H), 6.77 (t, 1H), 7.11 (d, 2H), 7.19 (t, 2H), 9.02 (s, 1H), 10.7 (bs, 1H) was prepared in accordance with the general method of example 1 from 4-ethyl-cyclohexanone and 1-phenyl-1,3,8-triaza-spiro[4.5]decan-4-one.

### Example 93

### (2RS,4aSR,8aRS)-8-(Decahydro-naphthalen-2-yl)-1-phenyl-1,3,8-triaza-spiro[4.5]decan-4-one hydrochloride (1:1)

The tide compound, m.p. >250°C and NMR (DMSO-d₆): 1.1- 2.0 (m, 18H), 3.02 (t, 2H), 3.2 (bs, 1H), 3.45 (m, 2H), 3.70 (m, 2H), 4.62 (s, 2H), 6.78 (t, 1H), 7.10 (d, 2H), 7.19 (t, 2H), 9.03 (s, 1H), 10.6 (bs, 1H) was prepared in accordance with the general methods used to prepare example 67 but from 1-(2RS,4aSR,8aRS-decahydro-naphtalen-2-yl)-piperidin-4-one.

### Example 94

### 1:1-mixture of cis and trans 4-Isopropyl-1-(4-oxo-1-phenyl-1,3,8-triazaspiro[4.5]dec-8-yl)-cyclohexanecarbonitrile

4-Isopropyl-cyclohenanone (1.4g) and 1-phenyl-1,3,8-triazaspiro[4.5]decane-4-one were dissolved in 25 ml of acetic acid. To this was added, via syringe under argon, 1.25 ml of trimethylsilyl-cyanide. The mixture was stirred for 18 h at room temperature after which another 1.25 ml of trimethylsilyl-cyanide was added. After 50 h at room temperature, the reaction mixture was poured on ice, adjusted to pH 9 using 30% aqueous ammonium hydroxide and extracted three times with ethyl acetate. The combined organic layers were dried over sodium sulfate, filtered and evaporated to yield the title compound, 1.35g, as a white powder, m.p. 213-215°C, MS: m/e = 381(M+).

### Example 95

### 1:1-mixture of cis and trans 8-(4-Isopropyl-1-methyl-cyclohexyl)-1-phenyl-1,3,8-triazaspiro[4.5]decane-4-one hydrocloride (1:1)

The 1:1-mixture of cis and trans 4-isopropyl-1-(4-oxo-1-phenyl-1,3,8-triazaspiro[4.5]dec-8-yl)-cyclohexanecarbonitrile (0.65 mmol) was dissolved in 15 ml dry tetrahydrofurane under argon. To this was added, at room temperature, 270 mL of a 3N solution of methylmagnesium chloride in tetrahydrofurane at a rate that the internal temperature did not raise above 25°C. After 15 h stirring at room temperature, the reaction was quenched by addition of saturated aqueous ammonium chloride, then adjusted to pH 11 using aqueous sodium hydroxide and extracted with ethyl acetate. The combined organic layers were dried over sodium sulfate, filtered, evaporated and chromatographed on silica gel using ethyl acetate:cyclohexane 2:1 as the eluent to give the title compound, 168 mg as an off-white powder. The hydrochloride had m.p. > 250°C (decomp.), MS: m/e = 370 (M+).

### Example 96

### 1:1-mixture of cis and trans 8-(1,4-Diisopropyl-cyclohexyl)-1-phenyl-1,3,8-triazaspiro[4.5]decane-4-one hydrocloride (1:1)

The 1:1-mixture of cis and trans 4-isopropyl-1-(4-oxo-1-phenyl-1,3,8-triazaspiro[4.5]dec-8-yl)-cyclohexanecarbonitrile (1.5 mmol) was dissolved in 35 ml dry tetrahydrofurane under argon. This was added, at room temperature, to a freshly prepared solution of isopropylmagnesiumbromide (15 mmol) in tetrahydrofurane at a rate that the internal temperature did not raise above 25°C. After 15 h stirring at room temperature and 3 h at 70°C, the reaction was quenched by addition of saturated aqueous ammonium chloride, then adjusted to pH 11 using aqueous sodium hydroxide and extracted with ether. The combined organic layers were dried over sodium sulfate, filtered, evaporated to give the title compound, 265 mg as an off-white powder. The hydrochloride had m.p. > 245°C (decomp.), MS: m/e = 398 (M+).

### Example 97

### (RS)-2-Butyl-1-(4-chlorophenyl)-8-cyclodecyl-1,3,8-triazaspiro[4.5]decan-4-one hydrochloride 1:1

The title compound, slightly yellow powder, m.p. 263.3-264.6, MS: m/e = 461 (M+), was prepared in accordance to general example 53 from 8-benzyl-1-(4-chlorophenyl)-1,3,8-triazaspiro[4.5]dec-2-en-4-one, butyl-magnesiumchloride and cyclodecanone.

### Example 98

### (RS)-2-Butyl-1-phenyl-8-cyclodecyl-1,3,8-triazaspiro[4.5]decan-4-one hydrochloride 1:1

The title compound, white powder, m.p. 275.9-276.8, MS: m/e = 426 (M⁺), was prepared in accordance to general example 53 from 8-benzyl-1-phenyl-1,3,8-triazaspiro[4.5]dec-2-en-4-one, butyl-magnesiumchloride and cydodecanone.

### Example 99

### Mixture of 8-(cis-4-methylcyclohexyl)-1-(4-methylphenyl)-1,3,8-triazaspiro[4.5]decan-4-one hydrochoride 1:1 and 8-(trans-4-methylcyclohexyl)-1-(4-methylphenyl)-1,3,8-triazaspiro[4.5]decan-4-one hydrochoride 1:1

The tide compound, white powder, m.p. > 283°C, MS: m/e = 342 (M+1⁺), was prepared in accordance to general example 7 from 1-(4-methylphenyl)-1,3,8-triazaspiro[4.5]decan-4-one and 4-methyl-cyclohexanone.

### Example 100

### 8-cyclodecyl-1-(2-methoxyphenyl)-1,3,8-triazaspiro [4.5]decan-4-one hydrochloride 1:1

The title compound, white powder, m.p. > 228°C (decomp.), MS: m/e = 400 (M+1⁺), was prepared in accordance to general example 7 from 1-(2-methoxyphenyl)-1,3,8-triazaspiro[4.5]decan-4-one and cyclodecanone.

### Example 101

### 8-(Bicyclo-nonan-9-yl)-1-phenyl-1,3,8-triaza-spiro[4,5]decan-4-one hydrochloride (1:1)

The title compound, white solid, m.p. 309°C and MS: m/e = 354.3 (M+H+) was prepared in accordance with the general method of example 7 from bicyclo-nonan-9-one and 1-phenyl-1,3,8-triazaspiro[4,5] decan-4-one.

### Synthesis of Intermediates

### Example aa

### 1-(2RS,4aRS,8aSR-Decahydro-naphthalen-2-yl)-4-phenylamino-piperidine-4-carboxylic acid amide

1-(2RS,4aRS,8aSR-Decahydro-naphthalen-2-yl)-4-phenylamino-piperidine-4-carbonitrile (7 mmol) was added dropwise at room temperature to a mixture of acetic anhydride and formic acid (15 ml each). The mixture was stirred for 21 h at room temperature. Sodium hydroxide solution was added (pH = 7) and the mixture extracted with ethylacetate. Organic phases were pooled, dried with sodium sulfate and concentrated. Chromatography on silica gel (ethylacetate) yielded the formylated amine which was dissolved in *t*-butanol (60 ml). Ammonia (28%, 10 ml), water (10 ml) and hydrogen peroxide solution (33% in water, 8 ml) were added. The mixture was stirred for 3.5 h at room temperature, quenched with cold water (75 ml) and extracted with methylene chloride. Organic phases were pooled, dried with sodium sulfate and concentrated. Chromatography on silica gel (methylene chloride/methanol, 98:2) yielded the desired product as a solid. 1.5 g (55%) of 1-(2RS,4aRS,8aSR-decahydro-naphthalen-2-yl)-4-phenylamino-piperidine-4-carboxylic acid amide, m.p. >150°C dec. and MS: m/e = 356.3 (M+H⁺).

### Example ab

### 1-(2RS,4aRS,8aSR-Decahydro-naphthalen-2-yl)-4-phenylamino-piperidine-4-carbonitrile

1-(2RS,4aRS,8aSR-decahydro-naphthalen-2-yl)-piperidin-4-one (8 mmol) was dissolved in acetic acid (8 ml). Aniline (9 mmol) and trimethylsilylcyanide (8 mmol) were added and the mixture was stirred for 90 min at room temperature. The reaction mixture was poured into cold ammonia solution (water/28% ammonia, 70ml/30ml). The solution was adjusted to pH 10 and extracted with dichloromethane. Organic phases were pooled, dried with sodium sulfate and concentrated. Crystallization from diethylether yielded the desired product as a solid. 2.8 g (97%) of 1-(2RS,4aRS,8aSR-Decahydronaphthalen-2-yl)-4-phenylamino-piperidine-4-carbonitrile, m.p. 153-156 °C and MS: m/e = 338.3 (M+H⁺).

### Example ac

### 1-(2RS,4aRS,8aSR-decahydro-naphthalen-2-yl)-piperidin-4-one

(2RS,4aRS,8aSR)-Decahydro-2-naphthylamine (12 mmol) was dissolved in ethanol (30 ml). Potassium carbonate (7.4 mmol) and 1-ethyl-1-methyl-4-oxo-piperidinium iodide (19 mmol) dissolved in water (10 ml) were added and the mixture was refluxed for 1 h. Water was added, ethanol was removed in vacuo and the residue was extracted with ethylacetate. Organic phases were pooled, dried with sodium sulfate and concentrated. Chromatography on silica gel (ethylacetate) yielded the desired product as an oil. 2.0 g (71%) of 1-(2RS,4aRS,8aSR-decahydro-naphthalen-2-yl)-piperidin-4-one, MS: m/e = 235 (M⁺).

### Example ad

### 8-Benzyl-1-p-tolyl-1,3,8-triazaspiro[4.5]dec-2-en-4-one

1-Benzyl-4-piperidone (50 mMol, 9.46g) and 4-methyl-aniline (50 mMol, 5.36g) were dissolved in acetic acid (100 ml) and cooled to 0°C. Trimethylsilyl cyanide (100 mMol, 12.5 ml) was added at such a rate, that the temperature did not rise above 5°C. After 20 h at room temperature, the reaction mixture was poured on ice, adjusted to pH 10 using 30% aqueous ammonium hydroxide and extracted three times with dichloromethane. The combined organic layers were dried over sodium sulfate, filtered and evaporated to yield 4-(p-tolylamino)-1-phenylmethyl)-4-piperidine-carbonitrile (13.4g) as a slightly yellow powder which was recrystallized from cyclohexane. m.p. 116.5-117°C. 4-(p-tolyl-amino)-1-(phenylmethyl)-4-piperidine-carbonitrile (4.58 g) was dissolved in 25 ml conc. sulfuric acid and stirred for 70 h at room temperature. Then the reaction mixture was poured on ice and adjusted to pH 10 by addition of 30% aqueous ammonium hydroxide and extracted three times with dichloromethane. The combined organic layers were dried over sodium sulfate, filtered and evaporated to yield 4-(p-tolyl-amino)-1-(phenylmethyl)-4-piperidine-carboxamide as an off-white powder (5.11g), m.p. 167.5-169°C.

4-(p-tolyl-amino)-1-(phenylmethyl)-4-piperidine-carboxamide (1.62 g) was dissolved in 10 ml triethyl orthoformate and stirred at 150°C for 15 hours. After cooling to room temperature, the reaction mixture was filtered and the light brown powder washed with ether to yield 8-benzyl-1-p-tolyl-1,3,8-triazaspiro[4.5]dec-2-en-4-one (0.99g). M.p. 208-213°C.

### Example ae

### 8-Benzyl-1-(4-chloro-phenyl)-1,3,8-triazaspiro[4.5]dec-2-en-4-one

1-Benzyl-4-piperidone (95 mMol, 16.9 ml) and 4-chloro-aniline (95 mMol, 12.1 g) were dissolved in acetic acid (100 ml) and cooled to 0°C. Trimethylsilyl cyanide (180 mMol, 22 ml) was added at such a rate, that the temperature did not rise above 5°C. After 4 d at room temperature, the reaction mixture was poured on ice, adjusted to pH 10 using 30% aqueous ammonium hydroxide and extracted three times with dichloromethane. The combined organic layers were dried over sodium sulfate, filtered and evaporated to yield 4-(4-chlorophenyl-amino)-1-(phenylmethyl)-4-piperidine-carbonitrile (30.3 g) as a slightly yellow powder, m.p. 142-150°C.

4-(4-chloro-phenyl -amino)-1-phenylmethyl)-4-piperidine-carbonitrile (29.3 g) was dissolved in 150 ml conc. sulfuric acid and stirred for 1 h at room temperature and 1 h at 70°C. Then the reaction mixture was cooled to room temperature and poured on ice, adjusted to pH 10 by addition of 30% aqueous ammonium hydroxide and extracted three times with dichloromethane. The combined organic layers were dried over sodium sulfate, filtered and evaporated to yield 4-(4-chloro-phenyl -amino)-1-(phenylmethyl)-4-piperidine-carboxamide as an brownish oil which crystallized upon addition of 50 ml diethyl ether and 50 ml pentane. Off-white powder (11.84 g), m.p. 167-170°C.

4-(4-chloro-phenyl -amino)-1-(phenylmethyl)-4-piperidine-carboxamide (6.47 g) was dissolved in 31 ml triethyl orthoformate and stirred at 150°C for 10 d. After cooling to 0°C, the reaction mixture was filtered and the light brown powder washed with ether to yield 8-benzyl-1-4-chloro-phenyl-1,3,8-triazaspiro[4.5]dec-2-en-4-one (3.90 g). M.p. 217-219°C.

### Example af

### 8-Benzyl-1-(-3-methyl-phenyl)-1,3,8-triazaspiro[4.5]dec-2-en-4-one

1-Benzyl-4-piperidone (100 mMol, 17.9 ml) and 3-methyl-aniline (100 mMol, 10.6 ml) were dissolved in acetic acid (100 ml) and cooled to 0°C. Trimethylsilyl cyanide (200 mMol, 25.5 ml) was added at such a rate, that the temperature did not rise above 5°C. After 3 d at room temperature, the reaction mixture was poured on ice, adjusted to pH 10 using 30% aqueous ammonium hydroxide and extracted three times with ethyl acetate. The combined organic layers were dried over sodium sulfate, filtered and evaporated to 50 ml, then treated with 50 ml diethyl ether and 50 ml pentane to yield 4-(3-methyl-phenyl-amino)-1-(phenylmethyl)-4-piperidine-carbonitrile as a slightly yellow powder which was washed with ether and dried .15.48 g, m.p. 95.7-97.1°C.

4-(3-methyl -phenyl-amino)-1-(phenylmethyl)-4-piperidine-carbonitrile (15.48 g) was dissolved in 77 ml conc. sulfuric acid and stirred for 18 h at room temperature. After cooling to room temperature, the reaction mixture was poured on ice and adjusted to pH 10 by addition of 30% aqueous ammonium hydroxide and extracted three times with dichloromethane. The combined organic layers were dried over sodium sulfate, filtered and evaporated to yield off-white crystals of 4-(3-methyl-phenyl-amino)-1-(phenylmethyl)-4-piperidine-carboxamide (14.46), m.p. 106.1-108.6°C.

4-(3-methyl-phenyl-amino)-1-(phenylmethyl)-4-piperidine-carboxamide (12.94 g) was dissolved in 80 ml triethyl orthoformate and stirred at 150°C for 66 hours. After cooling to 0°C, the reaction mixture was filtered and the light brown powder washed with ether to yield 8-benzyl-1-(3-methyl -phenyl)-1,3,8-triazaspiro[4.5]dec-2-en-4-one (2.1g). M.p. 301.5-302.6°C.

### Example ag

### 8-benzyl-1-(-2-methyl-phenyl)-1,3,8-triazaspiro[4.5]dec-2-en-4-one

1-Benzyl-4-piperidone (100 mMol, 17.9 ml) and 2-methyl-aniline (100 mMol, 10.6 ml) were dissolved in acetic acid (100 ml) and cooled to 0°C. Trimethylsilyl cyanide (200 mMol, 25.5 ml) was added at such a rate, that the temperature did not rise above 5°C. After 3 d at room temperature, the reaction mixture was poured on ice, adjusted to pH 10 using 30% aqueous ammonium hydroxide and extracted three times with ethyl acetate. The combined organic layers were dried over sodium sulfate, filtered and evaporated to 50 ml and treated with 50 ml diethyl ether and 50 ml pentane to yield 4-(3-methyl-phenyl-amino)-1-phenylmethyl)-4-piperidine-carbonitrile as a slightly yellow powder which was washed with ether and dried .18.89 g, m.p. 103.6-105.7°C.

4-(2-methyl -phenyl-amino)-1-(phenylmethyl)-4-piperidine-carbonitrile (17.89 g) was dissolved in 88 ml conc. sulfuric acid and stirred for 3 d at room temperature. The reaction mixture was poured on ice and adjusted to pH 10 by addition of 30% aqueous ammonium hydroxide and extracted three times with dichloromethane. The combined organic layers were dried over sodium sulfate and evaporated to yield 4-(2-methyl-phenyl-amino)-1-(phenylmethyl)-4-piperidine-carboxamide (10.35 g) as a brown oil. MS: m/z = 324 (M+H)⁺

4-(2-methyl-phenyl-amino)-1-(phenylmethyl)-4-piperidine-carboxamide (10.35 g) was dissolved in 60 ml triethyl orthoformate and stirred at 150°C for 10 d. After cooling to 0°C, the reaction mixture was evaporated and the resulting brown oil was chromatographed on silica gel using dichloromethane : methanol 19 : 1 as eluent. 8-benzyl-1-(2-methyl -phenyl)-1,3,8-triazaspiro[4.5]dec-2-en-4-one (4.71 g) was obtained as a brown foam. MS: m/z = 334 (M+H)⁺

### Example A

Tablets of the following composition are manufactured in the usual manner:

| | mg/tablet |
|---|---|
| Active substance | 5 |
| Lactose | 45 |
| Corn starch | 15 |
| Microcrystalline cellulose | 34 |
| Magnesium stearate | 1 |
| Tablet weight | 100 |

### Example B

Capsules of the following composition are manufactured:

| | mg/capsule |
|---|---|
| Active substance | 10 |
| Lactose | 155 |
| Corn starch | 30 |
| Talc | 5 |
| Capsule fill weight | 200 |

The active substance, lactose and corn starch are firstly mixed in a mixer and then in a comminuting machine. The mixture is returned to the mixer, the talc is added thereto and mixed thoroughly. The mixture is filled by machine into hard gelatine capsules.

### Example C

Suppositories of the following composition are manufactured:

| | mg/supp. |
|---|---|
| Active substance | 15 |
| Suppository mass | 1285 |
| Total | 1300 |

The suppository mass is melted in a glass or steel vessel, mixed thoroughly and cooled to 45°C. Thereupon, the finely powdered active substance is added thereto and stirred until it has dispersed completely. The mixture is poured into suppository moulds of suitable size, left to cool, the suppositories are then removed from the moulds and packed individually in wax paper or metal foil.

## Claims

1. Compounds of the general formula 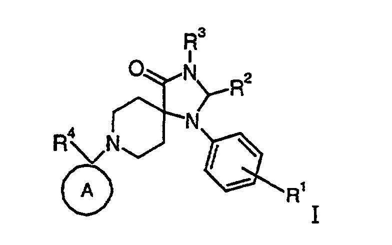 wherein
R¹ is hydrogen, (C₁₋₆)-alkyl, halogen, (C₁₋₆)-alkoxy, trifluoromethyl, (C₁₋₆)-alkyl-phenyl or (C₅₋₇)-cycloalkyl;
R² is hydrogen, (C₁₋₆)-alkyl, phenyl or (C₁₋₆)-alkyl-phenyl;
R³ is hydrogen, (C₁₋₆)-alkyl, benzyl, (C₁₋₆)-alkyl-phenyl, (C₁₋₆)-alkyl-diphenyl, triazinyl, cyanomethyl, (C₁₋₆)-alkyl-piperidinyl, (C₁₋₆)-alkyl-naphthyl, (C₅-₇)-cycloalkyl, (C₁₋₆)-alkyl-(C₅₋₇)-cycloalkyl, (C₁₋₆)-alkyl-pyridinyl, (C₁₋₆)-alkyl-morpholinyl, (C₁₋₆)-alkyl-dioxolanyl, (C₁₋₆)-alkyl-oxazolyl or (C₁₋₆)-alkyl-2-oxo-oxazoliding and wherein the ring systems may be substituted by additional (C₁₋₆)-alkyl, (C₁₋₆)-alkoxy, trifluoromethyl or phenyl, or -(CH₂)ₙC(O)O(C₁₋₆)- alkyl, -(CH₂)ₙC(O)NH₂,-(CH₂)ₙC(O)N[(C₁₋₆)-alkyl]₂, -(CH₂)ₙOH or -(CH₂)ₙC(O)NHCH₂C₆H₅;
R⁴ is hydrogen, (C₁₋₆)-alkyl or nitrilo;
A is a ring system, consisting of
(a) (C₅₋₁₅)-cycloalkyl, which maybe in addition to R⁴ optionally substituted by (C₁₋₆)-alkyl, trifluoromethyl, (C₅₋₇)-cycloalkyl, spiro-undecan-alkyl or by 2-norbornyl, or is one of the following groups 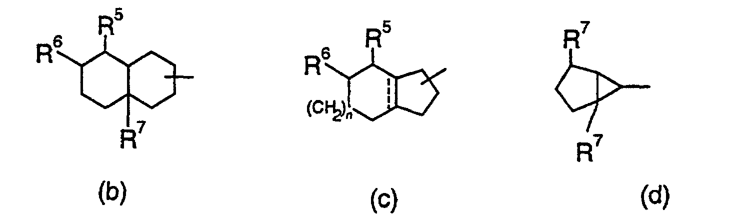 dodecahydro-acenaphthylen-1yl (e), bicyclo[6.2.0]dec-9-yl (f) and bicyclononan-9-yl (g);
and wherein
R⁵ and R⁶ are hydrogen, (C₁₋₆)-alkyl or taken together and with the carbon atoms to which they are attached form a phenyl ring;
R⁷ is hydrogen or (C₁₋₆)-alkyl;
the dotted line represents an optional bond and
n is 1 to 4;
and pharmaceutically acceptable acid addition salts thereof.

2. A compound according to claim 1, wherein A is an unsubstituted decahydro-naphthalenyl group of formula (b) and R¹-R³ are hydrogen, or R¹ and R² are hydrogen and R³ is methyl.

3. A compound according to claim 2 which is
8-(decahydro-naphthalen-2-yl)-1-phenyl-1,3,8-triaza-spiro[4.5]decan-4-one;
8-(decahydro-naphthalen-2-yl)-3-methyl-1-phenyl-1,3,8-triaza-spire[4.5] decan-4-one;
a mixture of (2RS,4aRS,8aSR)- and (2SR,4aRS,8aSR)-8-(decahydro-naphthalen-2-yl)-1-phenyl-1,3,8-triaza-spire[4.5]decan-4-one or
(2RS,4aSR,8aRS)-8-(Decahydro-naphthalen-2-yl)-1-phenyl-1,3,8-triaza-spiro [4.5] decan-4-one.

4. A compound according to claim 1, wherein A is cyclohexyl, optionally substituted by methyl or isopropyl, cyclodecyl or cyclononyl as defined under (a) and R¹-R³ are hydrogen.

5. A compound according to claim 4 which is
cis-8-(4-methyl-cyclohexyl)-1-phenyl-1,3,8-triaza-spiro [4.5]decan-4-one;
8-cyclodecyl-1-phenyl-1,3,8-triaza-spiro[4.5]decan-4-one;
8-cyclononyl-1-phenyl-1,3,8-triaza-spiro[4.5]decan-4-one or
cis-8-(4-isopropyl-cyclohexyl)-1-phenyl-1,3,8-triaza-spiro[4.5]decan-4-one.

6. A compound according to claim 1, wherein A is cyclodecyl, R¹ is hydrogen or methyl, R² is hydrogen or phenyl and R³ is hydrogen or cyanomethyl.

7. A compound according to claim 6 which is
(R,S)-8-cyclodecyl-1-(3-methyl-phenyl)-2-phenyl-1,3,8-triaza-spiro[4,5] decan-4-one or
(8-cyclodecyl-4-oxo-1-phenyl-1,3,8-triaza-spiro[4,5] dec-3-yl)-acetonitrile.

8. A compound according to claim 1, wherein A is an octahydro-indene group as defined under (c) and R¹-R³ are hydrogen.

9. A compound according to claim 8 which is 8-(cis-octahydro-inden-2-yl)-1-phenyl-1,3,8-triaza-spiro[4.5] decan-4-one.

10. A compound according to claim 1, wherein A is as defined under (f) and R¹-R³ are hydrogen.

11. A compound according to claim 10 which is 8-(cis-bicyclo [6.2.0] dec-9-yl)-1-phenyl-1,3,8-triaza-spiro [4,5]decan-4-one.

12. A medicament containing one or more compounds as claimed in any one of claims 1-11 and pharmaceutically acceptable excipients.

13. A medicament according to claim 12 for the treatment of diseases related to the Orphanin FQ (OFQ) receptor, which include memory and attention deficits, psychiatric, neurological and physiological disorders, such as anxiety and stress disorders, depression, trauma, memory loss due to Alzheimer's disease or other dementias, epilepsy and convulsions, acute and/or chronic pain conditions, and symptoms of addictive drug withdrawal, control of water balance, Na⁺ excretion, arterial blood pressure disorders and metabolic disorders such as obesity.

14. A process for preparing a compound of formula I as defined in claim 1, which process comprises
a) reductively aminating a compound of formula 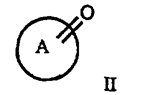 with a compound of formula 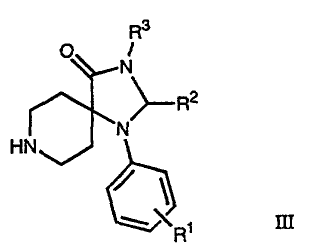 or, if desired, adding KCN to the mixture of a compound of formula II and III to get a compound of formula I, wherein R⁴ is hydrogen or nitrilo, respectively, and wherein A and R¹-R³ have the significances given above, or
b) cyclizing and reducing a compound of formula 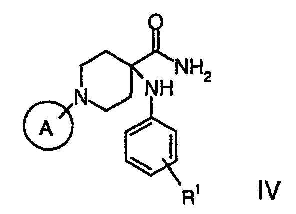 to give a compound of formula 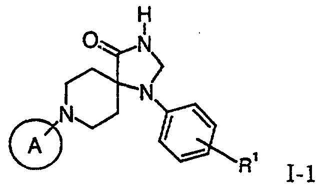 wherein A and R¹ have the significances given above, or
c) reacting a compound of formula 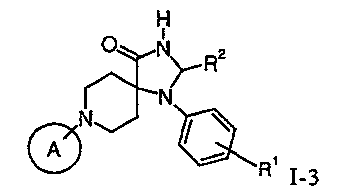 with a compound of formula
R³X
wherein R³ is described as above, but different from hydrogen and X is halogen, to give a compound of formula 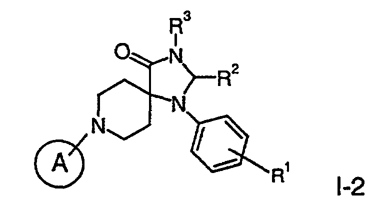 or
d) alkylating or benzylating a compound of formula I, wherein R³ is hydrogen, and, if desired,
e) converting a racemic mixture into its enantiomeric component thus obtaining optically pure compounds, and/or,if desired,
f) converting the compound of formula I obtained into a pharmaceutically acceptable acid addition salt.

15. The use of a compound claimed in any one of claims 1-11 for the manufacture of a medicament useful in the treatment of diseases related to the Orphanin FQ (OFQ) receptor, which include memory and attention deficits, psychiatric, neurological and physiological disorders, such as anxiety and stress disorders, depression, trauma, memory loss due to Alzheimer's disease or other dementias, epilepsy and convulsions, acute and/or chronic pain conditions, and symptoms of addictive drug withdrawal, control of water balance, Na⁺ excretion, arterial blood pressure disorders and metabolic disorders such as obesity.

## Patentansprüche

1. Verbindungen der allgemeinen Formel 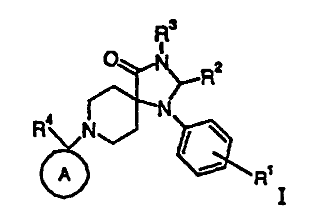 worin
R¹ Wasserstoff, (C₁₋₆)-Alkyl, Halogen, (C₁₋₆)-Alkoxy, Trifluormethyl, (C₁₋₆)-Alkyl-phenyl oder (C₅₋₇)-Cycloalkyl darstellt;
R² Wasserstoff, (C₁₋₆)-Alkyl, Phenyl oder (C₁₋₆)-Alkyl-phenyl darstellt;
R³ Wasserstoff, (C₁₋₆)-Alkyl, Benzyl, (C₁₋₆)-Alkyl-phenyl, (C₁₋₆)-Alkyl-diphenyl, Triazinyl, Cyanomethyl, (C₁₋₆)-Alkyl-piperidinyl, (C₁₋₆) -Alkyl-naphthyl, (C₅₋₇)-Cycloalkyl, (C₁₋₆)-Alkyl- (C₅₋₇)-cycloalkyl, (C₁₋₆)-Alkyl-pyridinyl, (C₁₋₆)-Alkyl-morpholinyl, (C₁₋₆)-Alkyl-dioxolanyl, (C₁₋₆)-Alkyl-oxazolyl oder (C₁₋₆)-Alkyl-2-oxo-oxazolidinyl darstellt und wobei die Ringsysteme durch zusätzliche (C₁₋₆)-Alkyl-, (C₁₋₆)-Alkoxy-, Trifluormethyl- oder Phenylgruppen substituiert sein können, oder -(CH₂)ₙC(O)O(C₁₋₆)-Alkyl, -(CH₂)ₙC(O)NH₂, -(CH₂)ₙC(O)N[(C₁₋₆)-Alkyl]₂, -(CH₂)ₙOH oder -(CH₂)ₙC(O)NHCH₂C₆H₅ darstellt;
R⁴ Wasserstoff, (C₁₋₆)-Alkyl oder Nitril darstellt;
A ein Ringsystem darstellt, bestehend aus
(a) (C₅₋₁₅)-Cycloalkyl, das zusätzlich zu R⁴ gegebenenfalls mit (C₁₋₆)-Alkyl, Trifluormethyl, (C₅₋₇)-Cycloalkyl, Spiroundecanalkyl oder mit 2-Norbornyl substituiert sein kann, oder eine der nachstehenden Gruppen 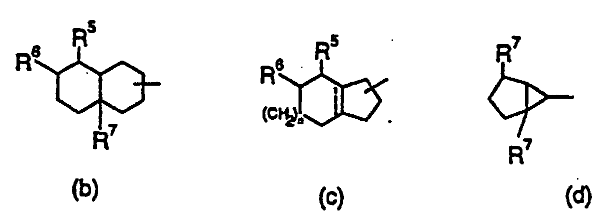 Dodecahydroacenaphthylen-1-yl (e), Bicyclo[6.2.0]dec-9-yl (f) und Bicyclononan-9-yl (g) darstellt,
und worin
R⁵ und R⁶ Wasserstoff, (C₁₋₆)-Alkyl darstellen oder zusammengenommen und mit den Kohlenstoffatomen, an die sie gebunden sind, einen Phenylring bilden;
R⁷ Wasserstoff oder (C₁₋₆)-Alkyl darstellt
und die Punktlinie eine mögliche Bindung wiedergibt und
n 1 bis 4 ist;
und pharmazeutisch verträgliche Säureadditionssalze davon.

2. Verbindung nach Anspruch 1, worin A eine unsubstituierte Decahydronaphthalinylgruppe der Formel (b) darstellt und R¹-R³ Wasserstoff darstellen oder R¹ und R² Wasserstoff darstellen und R³ Methyl darstellt.

3. Verbindung nach Anspruch 2, nämlich
8-(Decahydro-naphthalin-2-yl)-1-phenyl-1,3,8-triaza-spiro [4.5]decan-4-on;
8-(Decahydro-naphthalin-2-yl)-3-methyl-1-phenyl-1,3,8-triaza-spiro[4.5]decan-4-on;
ein Gemisch von (2RS,4aRS,8aSR)- und (2SR,4aRS,8aSR)-8-(Decahydro-naphthalin-2-yl)-1-phenyl-1,3,8-triaza-spiro-[4.5]decan-4-on oder
(2RS,4aSR,8aRS)-8-(Decahydro-naphthalin-2-yl)-1-phenyl-1,3,8-triaza-spiro[4.5]decan-4-on.

4. Verbindung nach Anspruch 1, worin A Cyclohexyl, gegebenenfalls substituiert mit Methyl oder Isopropyl, Cyclodecyl oder Cyclononyl, wie unter (a) definiert, darstellt und R¹-R³ Wasserstoff darstellen.

5. Verbindung nach Anspruch 4, nämlich
cis-8-(4-Methyl-cyclohexyl)-1-phenyl-1,3,8-triaza-spiro[4.5]decan-4-on;
8-Cyclodecyl-l-phenyl-1,3,8-triaza-spiro[4.5]decan-4-on;
8-Cyclononyl-1-phenyl-1,3,8-triaza-spiro[4.5]decan-4-on oder
cis-8-(4-Isopropyl-cyclohexyl)-1-phenyl-1,3,8-triaza-spiro [4.5]decan-4-on.

6. Verbindung nach Anspruch 1, worin A Cyclodecyl darstellt, R¹ Wasserstoff oder Methyl darstellt, R² Wasserstoff oder Phenyl darstellt und R³ Wasserstoff oder Cyanomethyl darstellt.

7. Verbindung nach Anspruch 6, nämlich
(R,S)-8-Cyclodecyl-1-(3-methyl-phenyl)-2-phenyl-1,3,8-triaza-spiro[4,5]decan-4-on oder
(8-Cyclodecyl-4-oxo-1-phenyl-1,3,8-triaza-spiro-[4,5]dec-3-yl)-acetonitril.

8. Verbindung nach Anspruch 1, worin A eine Octa-hydroinden-Gruppe, wie unter (c) definiert, darstellt und R¹-R³ Wasserstoff darstellen.

9. Verbindung nach Anspruch 8, nämlich
8-(cis-Octahydro-inden-2-yl)-1-phenyl-1,3,8-triaza-spiro[4.5]decan-4-on.

10. Verbindung nach Anspruch 1, worin A wie unter (f) definiert ist und R¹-R³ Wasserstoff darstellen.

11. Verbindung nach Anspruch 10, nämlich
8-(cis-Bicyclo[6.2.0]dec-9-yl)-1-phenyl-1,3,8-triaza-spiro[4,5]decan-4-on.

12. Arzneimittel, enthaltend eine oder mehrere Verbindungen nach einem der Ansprüche 1-11 und pharmazeutisch verträgliche Exzipienten.

13. Arzneimittel nach Anspruch 12 zur Behandlung von Erkrankungen, die mit dem Orphanin FQ (OFQ)-Rezeptor in Beziehung stehen, die Gedächtnis- und Aufmerksamkeitsdefizite, psychiatrische, neurologische und physiologische Erkrankungen, wie Beklemmung und Stresserkrankungen, Depression, Trauma, Gedächtnisverlust aufgrund Alzheimer-Krankheit oder anderer Demenz, Epilepsie und Konvulsionen, akute und/oder chronische Schmerzzustände und Symptome von Arzneimittelsuchtentzug, Regelung des Wasserhaushalts, Na⁺-Ausscheidung, arterielle Blutdruckerkrankungen und metabolische Erkrankungen, wie Fettsucht, einschließen.

14. Verfahren zur Herstellung einer Verbindung der Formel I, wie in Anspruch 1 definiert, wobei das Verfahren umfasst
a) reduktives Aminieren einer Verbindung der Formel 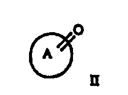 mit einer Verbindung der Formel 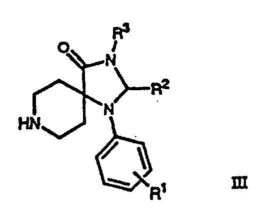 oder, falls erwünscht, Zugeben von KCN zu dem Gemisch einer Verbindung der Formel II und III zur Gewinnung einer Verbindung der Formel I, worin R⁴ Wasserstoff bzw. Nitril darstellt, und worin A und R¹-R³ die vorstehend angegebenen Bedeutungen aufweisen, oder
b) Cyclisieren und Reduzieren einer Verbindung der Formel 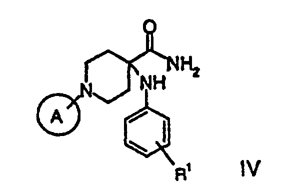 unter Gewinnung einer Verbindung der Formel 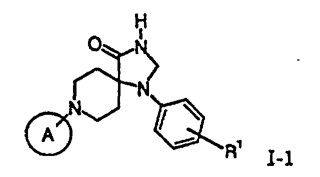 worin A und R¹ die vorstehend angegebenen Bedeutungen aufweisen, oder
c) Umsetzen einer Verbindung der Formel 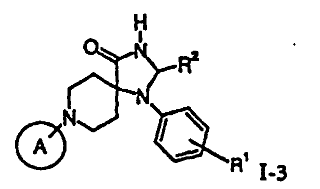 mit einer Verbindung der Formel
R³X,
worin R³ wie vorstehend beschrieben ist, jedoch sich von Wasserstoff unterscheidet, und X Halogen darstellt unter Gewinnung einer Verbindung der Formel 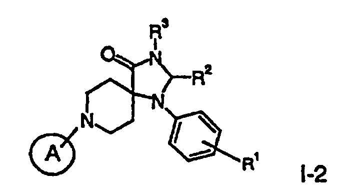 oder
d) Alkylieren oder Benzylieren einer Verbindung der Formel I, worin R³ Wasserstoff darstellt und, falls erwünscht,
e) Umwandeln eines racemischen Gemisches in seine enantiomere Komponente, wodurch optisch reine Verbindungen erhalten werden und/oder, falls erwünscht,
f) Umwandeln der erhaltenen Verbindung der Formel I in ein pharmazeutisch verträgliches Säureadditionssalz.

15. Verwendung einer Verbindung nach einem der Ansprüche 1-11 zur Herstellung eines Arzneimittels, das bei der Behandlung von Erkrankungen nützlich ist, die mit dem Orphanin FQ (OFQ)-Rezeptor in Beziehung stehen, die Gedächtnis- und Aufmerksamkeitsdefizite, psychiatrische, neurologische und physiologische Erkrankungen, wie Beklemmung und Stresserkrankungen, Depression, Trauma, Gedächtnisverlust aufgrund Alzheimer-Krankheit oder anderer Demenz, Epilepsie und Konvulsionen, akute und/oder chronische Schmerzzustände und Symptome von Arzneimittelsuchtentzug, Regelung des Wasserhaushalts, Na⁺-Ausscheidung, arterielle Blutdruckerkrankungen und metabolische Erkrankungen, wie Fettsucht, einschließen.

## Revendications

1. Composés de formule générale : 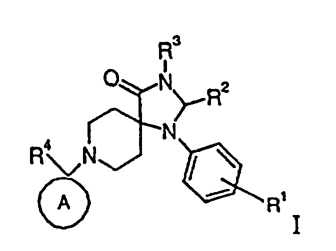 dans laquelle :
R¹ est un atome d'hydrogène ou un groupe alkyle en C₁₋₆, halogène, alcoxy en C₁₋₆, trifluorométhyle, alkyl(en C₁₋₆)phényle ou cycloalkyle en C₅₋₇ ;
R² est un atome d'hydrogène ou un groupe alkyle en C₁₋₆, phényle ou alkyl(en C₁₋₆)phényle ;
R³ est un atome d'hydrogène ou un groupe alkyle en C₁₋₆, benzyle, alkyl(en C₁₋₆)phényle, alkyl(en C₁₋₆)diphényle, triazinyle, cyanométhyle, alkyl(en C₁₋₆)pipéridinyle, alkyl(en C₁₋₆)naphtyle, cycloalkyle en C₅₋₇, alkyl(en C₁₋ ₆)-cycloalkyle(en C₅₋₇), alkyl(en C₁₋₆)pyridinyle, alkyl(en C₁₋₆)-morpholinyle, alkyl(en C₁₋₆)dioxolanyle, alkyl(en C₁₋₆)oxazolyle ou alkyl(en C₁₋₆)-2-oxo-oxazolidinyle et dans lequel les systèmes cycliques peuvent être substitués par des groupes alkyle en C₁₋₆, alcoxy en C₁₋₆, trifluorométhyle ou phényle, ou -(CH₂)ₙC(O)O-alkyle(en C₁₋₆), -(CH₂)ₙC(O)NH₂, -(CH₂)ₙC(O)N[alkyle(en C₁₋₆)]₂, -(CH₂)ₙOH ou -(CH₂)ₙC(O)NH-CH₂C₆H₅;
R⁴ est un atome d'hydrogène ou un groupe alkyle en C₁₋₆ ou nitrilo ;
A est un système cyclique constitué de
(a) cycloalkyle en C₅₋₁₅, qui peut être éventuellement, en plus de R⁴, substitué par un groupe alkyle en C₁₋₆, trifluorométhyle, cycloalkyle en C₅₋₇, spiroundécanalkyle ou par un 2-norbornyle, ou encore est l'un des groupes suivants : 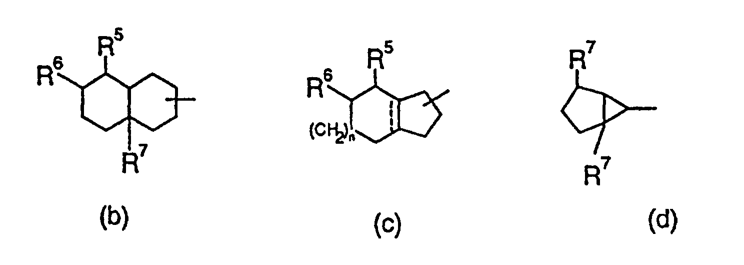 dodécahydro-acénaphtylén-1-yle (e), bicyclo[6.2.0]déc-9-yle (f) et bicyclononan-9-yle (g) ;
et dans laquelle
R⁵ et R⁶ sont des atomes d'hydrogène ou des groupes alkyle en C₁₋₆ ou bien forment, pris conjointement et avec les atomes de carbone auquel ils sont fixés, un noyau phényle,
R⁷ est un atome d'hydrogène ou un groupe alkyle en C₁₋₆; le trait pointillé représente une liaison facultative et
n vaut de 1 à 4 ;
et leurs sels d'addition d'acide pharmaceutiquement acceptables.

2. Composé selon la revendication 1, dans lequel A est un groupe décahydro-naphtalényle non substitué de formule (b) et R¹-R³ sont des atomes d'hydrogène, ou R¹ et R² sont des atomes d'hydrogène et R³ est un groupe méthyle.

3. Composé selon la revendication 2 qui est :
◆ la 8-(décahydronaphtalén-2-yl)-1-phényl-1,3,8-triazaspiro[4.5]décan-4-one
◆ la 8-(décahydronaphtalén-2-yl)-3-méthyl-1-phényl-1,3,8-triazaspiro[4.5]-décan-4-one
◆ un mélange de (2RS,4aRS,8aSR)- et de (2SR,4aRS,8aSR)-8-(décahydro-naphtalén-2-yle)-1-phényl-1,3,8-triazaspiro[4.5]décan-4-one ou
◆ la (2RS,4aSR,8aRS)-8-(décahydronaphtalén-2-yl)-1-phényl-1,3,8-triazaspiro-[4.5]décan-4-one.

4. Composé selon la revendication 1, dans lequel A est un groupe cyclohexyle, éventuellement substitué par un groupe méthyle ou isopropyle, cyclodécyle ou cyclononyle tel que défini en (a) et R¹-R³ sont des atomes d'hydrogène.

5. Composé selon la revendication 4 qui est :
◆ la cis-8-(4-méthylcyclohexyl)-1-phényl-1,3,8-triazaspiro[4.5]décan-4-one
◆ la 8-cyclodécyl-1-phényl-1,3,8-triazaspiro[4.5]décan-4-one
◆ la 8-cyclononyl-1-phényl-1,3,8-triazaspiro[4.5]décan-4-one ou
◆ la cis-8-(4-isopropylcyclohexyl)-1-phényl-1,3,8-triazaspiro[4.5]décan-4-one.

6. Composé selon la revendication 1 dans lequel A est un groupe cyclodécyle, R¹ est un atome d'hydrogène ou un groupe méthyle, R² est un atome d'hydrogène ou un groupe phényle et R³ est un atome d'hydrogène ou un groupe cyanométhyle.

7. Composé selon la revendication 6 qui est :
◆ la (R,S)-8-cyclodécyl-1-(3-méthylphényl)-2-phényl-1,3,8-triazaspiro[4.5]-décan-4-one ou
◆ le (8-cyclodécyl-4-oxo-1-phényl-1,3,8-triazaspiro[4.5]déc-3-yl)acétonitrile.

8. Composé selon la revendication 1, dans lequel A est un groupe octahydro-indène tel que défini en (c) et R¹-R³ sont des atomes d'hydrogène.

9. Composé selon la revendication 8 qui est la 8-(cis-octahydroindén-2-yl)-1-phényl-1,3,8-triazaspiro[4.5]décan-4-one.

10. Composé selon la revendication 1, dans lequel A est tel que défini en (f) et R¹-R³ sont des atomes d'hydrogène.

11. Composé selon la revendication 10 qui est la 8-(cis-bicyclo[6.2.0]déc-9-yl)-1-phényl-1,3,8-triazaspiro[4.5]décan-4-one.

12. Médicament contenant un ou plusieurs composés selon l'une quelconque des revendications 1-11 et des excipients pharmaceutiquement acceptables.

13. Médicament selon la revendication 12 pour le traitement de maladies liées au récepteur d'Orphanine FQ (OFQ), qui incluent les défauts de mémoire et d'attention, les troubles psychiatriques, neurologiques et physiologiques, tels que les troubles de l'anxiété et du stress, la dépression, le traumatisme, la perte de mémoire due à la maladie d'Alzheimer ou à d'autres démences, l'épilepsie et les convulsions, les affections douloureuses aigus et/ou chroniques, et les symptômes de manque des toxicomanes, la régulation du bilan hydrique, l'excrétion de Na⁺, les troubles de la tension artérielle et les troubles métaboliques tels que l'obésité.

14. Procédé de préparation d'un composé de formule 1 tel que défini dans la revendication 1, lequel procédé comprend les étapes consistant à :
a) soumettre à une amination réductrice un composé de formule : 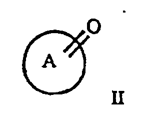 avec un composé de formule 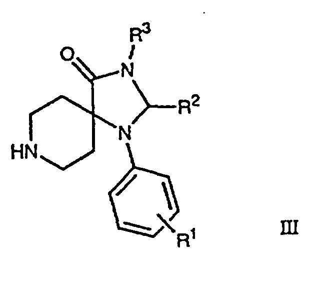 ou, le cas échéant, ajouter KCN au mélange d'un composé de formule II et III pour obtenir un composé de formule I, dans laquelle R⁴ est un atome d'hydrogène ou un groupe nitrilo, respectivement, et dans laquelle A et R¹-R³ ont les définitions données ci-dessus, ou
b) cycliser et réduire un composé de formule : 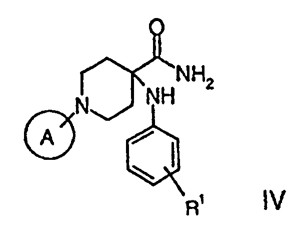 pour obtenir un composé de formule : 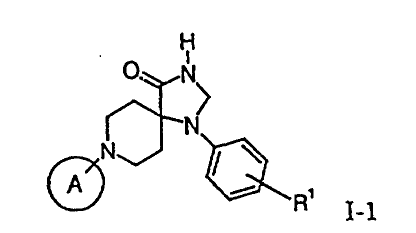 dans laquelle A et R¹ ont les significations indiquées ci-dessus, ou
c) faire réagir un composé de formule : 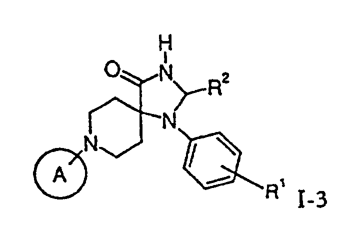 avec un composé de formule
R³X
dans laquelle R³ est tel que décrit ci-dessus, mais est différent d'un hydrogène, et X est un halogène, pour obtenir un composé de formule : 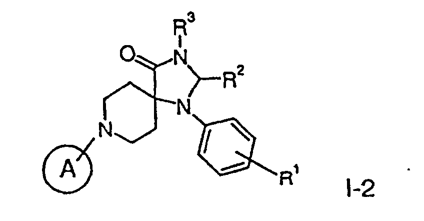 ou
d) alkyler ou benzyler un composé de formule I, dans laquelle R³ est un hydrogène et, le cas échéant,
e) convertir un mélange racémique en son constituant énantiomère pour obtenir des composés optiquement purs, et/ou, le cas échéant,
f) convertir le composé de formule I obtenu en un sel d'addition d'acide pharmaceutiquement acceptable.

15. Utilisation d'un composé selon l'une quelconque des revendications 1-11 pour la fabrication d'un médicament utile dans le traitement de maladies liées au récepteur d'Orphanine FQ (OFQ), qui incluent les défauts de mémoire et d'attention, les troubles psychiatriques, neurologiques et physiologiques, tels que les troubles de l'anxiété et du stress, la dépression, le traumatisme, la perte de mémoire due à la maladie d'Alzheimer ou à d'autres démences, l'épilepsie et les convulsions, les affections douloureuses aigus et/ou chroniques, et les symptômes de manque des toxicomanes, la régulation du bilan hydrique, l'excrétion de Na⁺, les troubles de la tension artérielle et les troubles métaboliques tels que l'obésité.
